(19) Europäisches Patentamt
European Patent Office
Office européen des brevets

(11) **EP 4 659 732 A1**

(12) **EUROPEAN PATENT APPLICATION**
published in accordance with Art. 153(4) EPC

(43) Date of publication:
**10.12.2025 Bulletin 2025/50**

(21) Application number: **23919486.3**

(22) Date of filing: **08.12.2023**

(51) International Patent Classification (IPC):
***A61K 8/35** (2006.01)* ***A61Q 19/00** (2006.01)*
***A61Q 19/08** (2006.01)*

(52) Cooperative Patent Classification (CPC):
**A61K 8/35; A61Q 19/00; A61Q 19/08**

(86) International application number:
**PCT/CN2023/137492**

(87) International publication number:
**WO 2024/159923 (08.08.2024 Gazette 2024/32)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.01.2023 CN 202310093262**

(71) Applicant: **Shiseido Company, Ltd.**
**Chuo-ku**
**Tokyo 104-0061 (JP)**

(72) Inventors:
- **YANG, Dandan**
  **Shanghai 200444 (CN)**
- **WANG, Yong**
  **Shanghai 200444 (CN)**
- **ZHAO, Qi**
  **Shanghai 200444 (CN)**
- **NISHIJIMA, Yoshihito**
  **Shanghai 201203 (CN)**
- **HU, Zhenlin**
  **Shanghai 200444 (CN)**
- **LI, Tan**
  **Shanghai 201203 (CN)**

(74) Representative: **Ter Meer Steinmeister & Partner Patentanwälte mbB**
**Nymphenburger Straße 4**
**80335 München (DE)**

(54) **EXTERNAL SKIN BARRIER FUNCTION REPAIRING AGENT**

(57) The present invention relates to a novel external skin barrier function repair agent, which not only can promote the repair of damaged skin barrier, but also can be expected to improve skin barrier dysfunction caused by the use of glucocorticoids and/or psychological stress. The skin barrier function repair agent achieves skin barrier repair through any of the following promoting actions: promoting the proliferation of keratinocytes, promoting the migration of keratinocytes, promoting the expression of HAS-2 in keratinocytes, and promoting the expression of HBD-2 in keratinocytes.

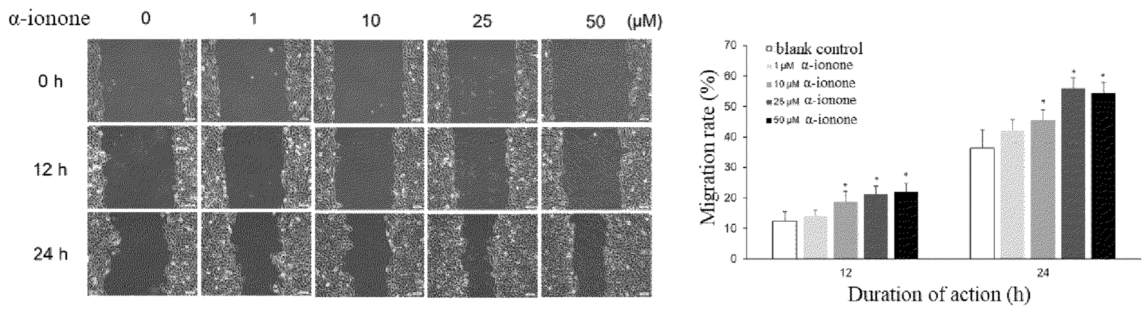


**Figure 2**

EP 4 659 732 A1

## Description

## Technical Field

The present invention relates to a novel external skin barrier function repair agent, which not only can promote the repair of damaged skin barrier, but also can be expected to improve skin barrier dysfunction caused by the use of glucocorticoids and/or psychological stress. The skin barrier function repair agent achieves skin barrier repair through any of the following promoting actions: promoting the proliferation of keratinocytes, promoting the migration of keratinocytes, promoting the expression of hyaluronan synthase-2 (HAS-2) in keratinocytes, and promoting the expression of human β-defensin-2 (HBD-2) in keratinocytes.

## Background Art

The skin serves as the primary protective barrier between the body and the external environment, undertaking the crucial functions of preventing excessive loss of moisture from the body and blocking the invasion of harmful substances from the environment. This barrier function is predominantly provided by the epidermis. Located in the outermost layer of the skin, the epidermis is a stratified epithelial tissue that continuously renews itself. Keratinocytes (also known as basal cells) in the innermost basal layer of the epidermis continuously divide and proliferate to produce daughter cells for epidermal renewal, and as these newly generated daughter cells migrate towards the outer layers, they gradually differentiate into spinous and granular layer cells, eventually becoming corneocytes. The outermost stratum corneum continuously sheds through the process of desquamation, while underlying cells migrate upward to form a new stratum corneum. Therefore, maintaining the homeostasis of the epidermal barrier requires a balance in the proliferation, migration, and differentiation activities of keratinocytes.

As the outermost barrier of the body, the skin is constantly exposed to harmful external environmental factors, which can lead to tissue damage and barrier disruption. Following skin injury, a series of repair processes are typically immediately activated to restore skin barrier function. The repair of skin injury is a highly coordinated process involving various types of cells, extracellular matrix molecules, and regulatory factors. As the main cell type of the epidermis, keratinocytes play a key role in wound healing and the repair of epidermal barrier function. Once an injury occurs, keratinocytes at the wound edges are immediately activated, accelerating their migration and proliferation to facilitate re-epithelialization of the skin, which is crucial for rapid wound healing.

In addition to accelerating proliferation and migration, keratinocytes also promote barrier repair and maintenance of skin homeostasis by upregulating the expression of various functionally important genes such as hyaluronic acid synthase (HAS) and human β-defensin (HBD). HAS is the key enzyme for the synthesis of hyaluronic acid (HA) in skin cells. Keratinocytes can express three subtypes of HAS, namely HAS-1, HAS-2, and HAS-3, among which HAS-2 is the most abundantly expressed and is the most important enzyme for HA synthesis in keratinocytes. HA is the most abundant component of the extracellular matrix in skin tissue and is involved in processes such as tissue homeostasis maintenance, hydration, and wound repair. Substantial research evidence has demonstrated the crucial role of HA in the wound healing process. During wound healing, a large amount of HA is produced locally at the wound area, the locally produced HA and its catabolic products (medium- and low-molecular-weight HA) selectively activate the CD44 receptors on the surface of keratinocytes, which in turn mediate specific signaling pathways to regulate the proliferation, migration, and differentiation of keratinocytes, ultimately affecting wound healing. Studies have shown that overexpression of HAS-2 in keratinocytes accelerates cell migration, whereas knockdown of HAS-2 inhibits the migration of keratinocytes. Epidermal growth factor (EGF), keratinocyte growth factor (KGF), and all-trans retinoic acid (RA) can all increase the expression level of HAS-2 in keratinocytes, thereby enhancing the synthesis of HA. Moreover, this HAS-2-dependent increase in HA synthesis is directly associated with the roles of EGF, KGF, and RA in promoting keratinocyte migration, proliferation, and wound healing. It is evident that HA not only contributes to the composition of the epidermal extracellular matrix, but also plays a crucial role in the proliferation, migration, and differentiation of keratinocytes. Therefore, enhancing the expression of HAS-2 facilitates the rapid repair of skin injuries and the maintenance of barrier function homeostasis.

Human β-defensins (HBDs) are a group of cationic antimicrobial peptides composed of 12-50 amino acids that are produced by epithelial tissues such as the skin and respiratory tract, and possess antibacterial and antiviral activities, thereby constituting the first line of defense in the body's innate immune system. To date, four types of HBD have been identified in human skin: HBD-1, HBD-2, HBD-3, and HBD-4. The expression of HBD-2 in skin tissue is localized to the uppermost layer of the epidermis, and its expression level markedly increases in differentiated keratinocytes. Recent studies have also shown that HBD-2 mediates the protective effect of IL-1β-induced keratinocytes against proteases secreted by *Staphylococcus aureus,* suggesting that promoting endogenous HBD-2 expression may be beneficial for repairing the skin barrier and preventing the colonization of *Staphylococcus aureus* in inflammatory skin disease conditions. An increasing number of studies have shown that the functions of antimicrobial peptides extending far beyond their antimicrobial activity, and these peptides also play a role in wound repair and the maintenance of skin barrier

homeostasis through various pathways. After trauma, local epidermal cells upregulate HBD expression in response to stimulation by cytokines, growth factors, or bacteria. The increased HBD then activates signaling pathways such as MAPK, AKT, STAT1, and STAT3, as well as intracellular $Ca^{2+}$ signaling and EGFR phosphorylation, promotes keratinocyte proliferation and migration, thereby accelerating skin wound healing. In addition to influencing keratinocyte proliferation and migration, HBD also stimulates angiogenesis, promotes collagen production by dermal fibroblasts, downregulates the expression of matrix metalloproteinase-1, thereby facilitating extracellular matrix deposition to accelerate wound healing. Another study has found that HBD can improve skin barrier function by enhancing the expression of tight junction proteins in epidermal cells, and it selectively activates $Lgr6^+$ skin stem cells to promote epidermal renewal and damage repair, which suggests that defensins also play an important role in the maintenance of skin barrier homeostasis. Therefore, increasing the expression level of HBD in keratinocytes helps to improve the speed and quality of skin wound repair and regeneration.

Under normal circumstances, the skin itself has a strong ability to self-repair and can quickly repair minor injuries to restore the homeostasis of tissue structure and function. However, the repair process of skin injuries is often affected by various adverse factors, which can interfere with the normal progression of the repair process, delay the repair of injuries, lead to skin barrier dysfunction, and potentially trigger or exacerbate various skin diseases. Stress and the use of glucocorticoid drugs are two common adverse factors that affect skin injury repair. Stress is a non-specific response of the body triggered by excessive or harmful stimuli, arising from various physiological factors (such as trauma and pain) and psychological factors (stress), and can be divided into physiological stress and psychological stress. In particular, with the acceleration of the pace of life and the increase in competitive pressures from all sides, psychological stress has become an inevitable and frequent event in modern life. Psychological stress can lead to a variety of skin health issues, including reduced wound healing capacity, barrier dysfunction, abnormal skin immune function, decreased anti-infection ability, and even premature skin aging. Stress can trigger a series of complex physiological responses, primarily manifested as increased activity of the sympathetico-adrenomedullary system (SAM) and the hypothalamic-pituitary-adrenal axis (HPA axis). Activation of the SAM leads to a rapid increase in the levels of stress hormones such as adrenaline and noradrenaline, while increased activity of the HPA axis results in the release of corticotropin-releasing hormone (CRH) from the hypothalamus and the secretion of adrenocorticotropic hormone (ACTH) from the pituitary gland, which in turn stimulates the adrenal glands to secrete glucocorticoids (GC). In recent years, an increasing number of research evidence has shown that the skin is an important target organ for the aforementioned stress hormones. Excessively produced adrenaline and noradrenaline under stress conditions can directly act on the beta-2-adrenergic receptors on the surface of epidermal keratinocytes, inhibiting the migration of keratinocytes, inducing oxidative stress and DNA damage, and leading to skin barrier disruption, delayed wound healing, and skin aging. Glucocorticoids (GC) produced excessively under stress can also directly act on skin cells, inhibiting the expression of hyaluronic acid and lipid synthase, reducing the content of hyaluronic acid and lipid in skin tissue, negatively affecting the epidermal barrier, reducing hydration of the stratum corneum, and increasing transepidermal water loss. GC can also inhibit the proliferation and differentiation of keratinocytes in epidermal tissue, destroy the integrity and adhesion of the stratum corneum, and damage the epidermal barrier function. In addition, GC also inhibits the expression of antimicrobial peptides such as HBD by keratinocytes, impairing the skin's ability to resist infection. The use of exogenous glucocorticoids can produce adverse effects similar to those of endogenous GCs that are overproduced during stress. Epidermal dysfunction is common in patients who use glucocorticoids for a long time. Skin atrophy and barrier dysfunction are widely acknowledged adverse reactions associated with the topical application of glucocorticoid drugs. Studies have shown that the glucocorticoid drug dexamethasone can significantly inhibit the expression of HAS-2 and the synthesis of HA in skin fibroblasts and HaCaT keratinocytes at very low doses (1.5 nM, 150 nM, and 1.5 $\mu$M) and after a very short duration of action (3 h). Consistent with the above, human trials have shown that external application of dexamethasone ointment for a short treatment period (3 days) is enough to significantly lower the levels of HA in the skin tissue. In contrast, it takes 3 weeks of GC treatment to observe a reduction in the synthesis of type I and type III collagen in the skin tissue. This suggests that the downregulation of HAS-2 expression and the resulting decrease in HA synthesis may be the most critical initial steps in GC-induced skin atrophy.

Natural $\alpha$-ionone, a secondary metabolite of plants, possesses a violet floral scent and is widely found in various flowers, fruits such as cherries, raspberries, and grapes, as well as in tea and various plant essential oils. Synthetically produced $\alpha$-ionone has been extensively used as a fragrance ingredient in the food and daily chemical industries. In the *National Food Safety Standard for the Use of Food Additives* (GB2760-2014) promulgated in 2014, $\alpha$-ionone is classified as a synthetic flavoring for food, and the US FDA has also approved the use of $\alpha$-ionone as a flavoring. In addition to being used as a food additive, $\alpha$-ionone is widely used as a fragrance agent in cosmetics, perfumes, shampoos, soaps, and other toiletries, as well as in household cleaners and detergents. Statistics show that the global usage of $\alpha$-ionone is about 100 to 1,000 metric tons per year.

Our bodies and skin are often exposed to $\alpha$-ionone, but we know very little about its biological functions. A study has reported that $\alpha$-ionone may have anti-photoaging effects on the skin, finding that $\alpha$-ionone can activate the TGF-$\beta$-SMAD pathway in UV-irradiated Hs-68 human fibroblasts, induce collagen expression, but inhibit the MAPK-AP-1

signaling pathway and inhibit the expression of MMP1, MMP3 and MMP9 (Non-patent Literature 1, Molecules 2019, 24 (9), 1804). In another related study, α-ionone was shown to stimulate myogenesis *in vitro,* alleviating palmitate-induced atrophy of skeletal muscle myotubes, and increasing myotube diameter and length, fusion index, and cellular protein content. This study suggests that α-ionone is a potential candidate for enhancing the mass and strength of skeletal muscles. The aforementioned studies indicate that, in addition to its fragrance function, α-ionone may also possess biological functions that are beneficial to human health (Non-patent Literature 2, Food Funct. This journal is © The Royal Society of Chemistry 2019, published on February 5, 2019).

Additionally, a summary overview of the known biological activities of α-ionone can be found in the journal Science and Technology of Food Industry 2022; 43(20): 481-488 (Non-patent Literature 3).

Prior art literatures

Non-patent literatures:

1. Molecules 2019, 24 (9), 1804
2. Food Funct. This journal is © The Royal Society of Chemistry 2019, published on February 5, 2019
*3.* Science and Technology of Food Industry 2022; 43 (20): 481-488

**Description of the Invention**

**Technical Problem to be Solved by the Invention**

Based on the above understanding, it is known that skin barrier function is closely related to the proliferation and migration of keratinocytes, as well as the expression of HAS-2 and HBD-2 in keratinocytes. Based on the above understanding, the Inventors of the present invention have conducted in-depth research, starting from the search for substances that can regulate the proliferation, migration, and expression of HAS-2 and HBD-2 in keratinocytes, and are committed to finding new topical skin barrier function repair agents to improve the barrier function of damaged skin and repair skin barrier dysfunction caused by the use of glucocorticoids and/or psychological stress.

**Means for Solving the Technical Problem**

In order to find substances that can promote the proliferation and migration of keratinocytes as well as enhance the expression of HAS-2 and HBD-2 in keratinocytes, the Applicant went through in-depth research and a large number of experiments, and unexpectedly discovered that α-ionone can promote the proliferation and migration of keratinocytes, and promote the expression of HAS-2 and HBD-2 in the keratinocytes, and that the addition of α-ionone as an active ingredient in topical skin formulations significantly promotes the repair of the skin barrier. Based on this finding, the present invention was completed.

Specifically, the present invention includes the following solutions:

1. A skin barrier function repair agent for use in topical skin formulations, characterized in that it comprises α-ionone as an active ingredient acting on keratinocytes.
2. The skin barrier function repair agent according to item 1 above, characterized in that the α-ionone functions as a keratinocyte migration promoter.
3. The skin barrier function repair agent according to item 1 above, characterized in that the α-ionone functions as a keratinocyte proliferation promoter.
4. The skin barrier function repair agent according to item 1 above, characterized in that the α-ionone functions as a promoter of HAS-2 expression in keratinocytes.
5. The skin barrier function repair agent according to item 1 above, characterized in that the α-ionone functions as a promoter of HBD-2 expression in keratinocytes.
6. The skin barrier function repair agent according to item 1 above, characterized in that it is used as a repair agent for skin barrier dysfunction caused by the use of glucocorticoids and/or psychological stress.
7. The skin barrier function repair agent according to item 1 above, characterized in that the topical skin formulation is a cosmetic product.
8. Use of α-ionone in the preparation of cosmetics for promoting skin barrier repair, wherein the promotion of skin barrier repair is achieved through any of the following promoting actions: promoting the proliferation of keratinocytes, promoting the migration of keratinocytes, promoting the expression of HAS-2 in keratinocytes, and promoting the

expression of HBD-2 in keratinocytes.

9. Use of α-ionone in the preparation of cosmetics suitable for skin in a state with skin atrophy and/or skin barrier dysfunction caused by the use of glucocorticoids and/or psychological stress.

10. A keratinocyte proliferation promoter containing α-ionone as an active ingredient.

11. Use of α-ionone as a keratinocyte proliferation promoter.

12. Use of α-ionone in the preparation of topical skin formulations for promoting keratinocyte proliferation.

13. A keratinocyte migration promoter containing α-ionone as an active ingredient.

14. Use of α-ionone as a keratinocyte migration promoter.

15. Use of α-ionone in the preparation of topical skin formulations for promoting keratinocyte migration.

16. A promoter of HAS-2 expression in keratinocytes containing α-ionone as an active ingredient.

17. Use of α-ionone as a promoter of HAS-2 expression in keratinocytes.

18. Use of α-ionone in the preparation of topical skin formulations for promoting HAS-2 expression in keratinocytes.

19. A promoter of HBD-2 expression in keratinocytes containing α-ionone as an active ingredient.

20. Use of α-ionone as a promoter of HBD-2 expression in keratinocytes.

21. Use of α-ionone in the preparation of topical skin formulations for promoting HBD-2 expression in keratinocytes.

## Effects of the Invention

The Inventors of this invention have discovered for the first time that α-ionone can function as a promoter of keratinocyte migration, a promoter of keratinocyte proliferation, a promoter of HAS-2 expression in keratinocytes, and a promoter of HBD-2 expression in keratinocytes. It achieves skin barrier repair through any of the following promoting actions: promoting the proliferation of keratinocytes, promoting the migration of keratinocytes, promoting the expression of HAS-2 in keratinocytes, and promoting the expression of HBD-2 in keratinocytes. This makes it possible not only to address ordinary skin damage but also to repair skin atrophy and/or skin barrier dysfunction caused by the use of glucocorticoids and/or psychological stress.

## Brief Description of the Drawings

Figure 1 is a graph showing the effect of α-ionone on the proliferation of HaCaT cells.

Figure 2 is a graph showing the impact of α-ionone on the migration of HaCaT cells.

Figure 3 is a graph showing the impact of α-ionone on norepinephrine (NE)-induced inhibition of HaCaT cell migration.

Figure 4 is a graph showing the impact of α-ionone on the expression of HAS-2 in HaCaT cells.

Figure 5 is a graph showing the impact of α-ionone on the synthesis of hyaluronic acid (HA) in HaCaT cells.

Figure 6 is a graph showing the impact of α-ionone on the dexamethasone (DEX)-induced inhibition of HAS-2 expression.

Figure 7 is a graph showing the impact of α-ionone on the expression of HBD-2 in HaCaT cells.

Figure 8 is a graph showing the impact of α-ionone on the secretion of HBD-2 in HaCaT cells.

Figure 9 is a graph showing the impact of α-ionone on the dexamethasone (DEX)-induced inhibition of HBD-2 expression.

Figure 10 is a graph showing the transepidermal water loss (TEWL) values of the test areas in each experimental group.

Figure 11 is a graph showing the stratum corneum moisture content in the test areas of each experimental group.

Figure 12 is a graph showing the barrier repair rates of each experimental group.

Figure 13 is a graph showing the measured moisture content values of the stratum corneum of the subjects.

Figure 14 is a graph showing the change value in the stratum corneum moisture content of the subjects when compared with that before stripping.

Figure 15 is a graph showing the measured TEWL values of the test areas of the subjects.

Figure 16 is a graph showing the TEWL repair rates of the subjects.

## Detailed Description of the Embodiments

The following is a detailed description of the present invention. However, the invention is not limited to the specific embodiments described below. Various modifications and changes can be made to the invention without departing from the spirit and scope of the invention.

The term "topical skin formulation" as used in the present invention refers to any formulation designed for external application to the skin, including but not limited to cosmetics and perfumes, fragrances, solid perfumes, etc., such as

toners, softening water, moisturizing water, repair water, and other aqueous formulations; hydrating sprays, anti-wrinkle sprays, repair sprays, and other spray formulations; moisturizing emulsions, anti-wrinkle emulsions, repair emulsions, body lotions, hand lotions, and other emulsion formulations; moisturizing essences, anti-wrinkle essences, repair essences, and other essence formulations; moisturizing gels, anti-wrinkle gels, repair gels, and other gel formulations; facial creams, eye creams, moisturizing creams, anti-wrinkle creams, repair creams, massage creams, hand creams, body creams, and other creams or ointments; setting powders, loose powders, compact powders, talcum powders, and other powders.

Ionone, with the structural formula $C_{13}H_{20}O$ and a molecular weight of 192.3 g/mol, comprises three isomers: α-ionone, β-ionone, and γ-ionone. α-Ionone is also known as (3E)-4-(2,6,6-trimethylcyclohex-2-en-1-yl)but-3-en-2-one, cyclocitrylideneacetone, and irisone, etc. α-Ionone is a pale yellow transparent liquid with a melting point of -49°C and a boiling point of 126-128°C. The structural formula is as follows:

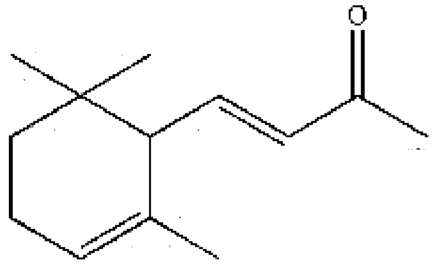

Ionone, a key aromatic component primarily found in raspberries *(Rubus idaeus,* Raspberry), yellow carrots (*Daucus carota subsp. Sativus,* Carrot), sweet almonds (*Prunus dulcis,* Almond), and mints (*Mentha,* Herb, Vanilla), and mainly produces floral aroma.

The method for obtaining α-ionone in the present invention is not particularly limited, and α-ionone may be isolated from plants containing the above-mentioned ionone, or chemically synthesized using a known production method, or a commercially available product may be used.

The skin barrier repair agent in the novel topical skin formulation of the present invention is characterized in that it comprises α-ionone as an active ingredient acting on keratinocytes.

The α-ionone, as a skin barrier repair agent, is present in the topical skin formulation in an amount of 0.0001% to 5% by mass, more preferably from 0.001% to 2% by mass, further preferably from 0.01% to 1.5% by mass, and particularly preferably from 0.1% to 1% by mass.

In addition to comprising α-ionone as an active ingredient for the skin barrier repair agent, the topical skin formulation of the present invention may include other additives such as excipients and carriers, and may appropriately use components commonly added to cosmetics as needed.

Components commonly added to cosmetics include, but are not limited to, for example, aqueous solvents, oily components, anionic surfactants, cationic surfactants, amphoteric surfactants, nonionic surfactants, water-soluble polymers, thickeners, preservatives, ultraviolet absorbers, metal ion sequestrants, amino acids, organic amines, polymeric emulsions, pH regulators, neutralizers, skin nutrients, vitamins, antioxidants, antioxidant synergists, fragrances, and the like.

As aqueous solvents, examples may include water, alcohol, moisturizers, and mixtures thereof.

Water used may be of the type employed in cosmetics, quasi-drugs, or similar applications, such as purified water, deionized water, or tap water. Depending on the intended purpose, the aqueous phase may further contain water-soluble alcohols.

The water-soluble alcohols can be exemplified by at least one selected from the group consisting of lower alcohols, polyols, polyol polymers, glycol alkyl ethers, glycol ether esters, glycerol monoalkyl ethers, sugar alcohols, monosaccharides, oligosaccharides, polysaccharides, and derivatives thereof, and the like.

The lower alcohols can be exemplified by ethanol, propanol, isopropanol, isobutanol, tert-butanol, and the like.

The polyols can be exemplified by diols (e.g., ethylene glycol, propylene glycol, trimethylene glycol, 1,2-butanediol, 1,3-butanediol, tetramethylene glycol, 2,3-butanediol, pentamethylene glycol, 2-butene-1,4-diol, hexanediol, octanediol, etc.); triols (e.g., glycerol, trimethylolpropane, etc.); tetraols (e.g., pentaerythritol, etc.); pentaols (e.g., xylitol, etc.); and hexaols (e.g., sorbitol, mannitol, etc.),.

The polyol polymers can be exemplified by diethylene glycol, dipropylene glycol, triethylene glycol, polypropylene glycol, tetraethylene glycol, diglycerol, triglycerol, tetraglycerol, and the like.

The glycol alkyl ethers can be exemplified by ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isopentyl ether, ethylene glycol benzyl ether, ethylene glycol isopropyl ether,

ethylene glycol dimethyl ether, ethylene glycol diethyl ether, ethylene glycol dibutyl ether, etc.; diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methyl ethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, and the like.

The glycol ether esters can be exemplified by ethylene glycol monomethyl ether acetate, ethylene glycol monoethyl ether acetate, ethylene glycol monobutyl ether acetate, ethylene glycol monophenyl ether acetate, ethylene glycol diadipic acid ester, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, and the like.

The glycerol monoalkyl ethers can be exemplified by chimyl alcohols, batyl alcohols, and selachyl alcohols, and the like.

The sugar alcohols can be exemplified by sorbitol, maltitol, maltotriose, mannitol, sucrose, erythritol, glucose, fructose, starch hydrolysis sugars, maltose, xylose, starch hydrolysis sugar reductol, and the like; tetrahydrofurfuryl alcohol; POE-tetrahydrofurfuryl alcohol; POP-butyl ether; POP•POE-butyl ether; tripropylene glycol ether; POP-glycerol ether; POP-glycerol ether phosphate; POP•POE-pentaerythritol ether, and the like.

The monosaccharides can be exemplified by at least one selected from the group consisting of trioses (e.g., D-glyceraldehyde, dihydroxyacetone, etc.), tetroses (e.g., D-erythrose, D-erythrulose, D-threose, erythritol, etc.), pentoses (e.g., L-arabinose, D-xylose, L-lyxose, D-arabinose, D-ribose, D-ribulose, D-xylulose, L-xylulose, etc.), hexoses (e.g., D-glucose, D-talose, D-altrose, D-galactose, D-fructose, L-galactose, L-mannose, D-tagatose, etc.), heptoses (e.g., heptoaldose, heptulose, etc.), octoses (e.g., octulose, etc.), deoxy sugars (e.g., 2-deoxy-D-ribose, 6-deoxy-L-galactose, 6-deoxy-L-mannose, etc.), amino sugars (e.g., D-glucosamine, D-galactosamine, sialic acid, muramic acid, etc.), uronic acids (e.g., D-glucuronic acid, L-guluronic acid, D-galacturonic acid, L-iduronic acid, etc.), and the like.

The monosaccharides can be exemplified by at least one selected from the group consisting of sucrose, umbelliferose, lactose, planteose, $\alpha,\alpha$-trehalose, and the like.

The polysaccharides can be exemplified by at least one selected from the group consisting of cellulose, quince seed, chondroitin sulfate, starch, galactan, dermatan sulfate, glycogen, gum arabic, heparan sulfate, hyaluronic acid, tragacanth gum, keratan sulfate, chondroitin, xanthan gum, mucopolysaccharide, guar gum, glucan, locust bean gum, succinoglycan, caraginic acid, and the like.

The other polyols can be exemplified by at least one selected from the group consisting of polyoxyethylene methyl glucoside (Glucam E-10), polyoxypropylene methyl glucoside (Glucam P-10), and the like.

The humectants can be exemplified by propylene glycol, glycerin, 1,3-butanediol, dipropylene glycol, xylitol, sorbitol, maltitol, chondroitin sulfate, hyaluronic acid, mucopolysaccharide, carolic acid, atelocollagen, sodium lactate, bile acid salts, dl-pyrrolidone carboxylate salts, epoxy alkane derivatives, short-chain soluble collagen, diglycerol (EO)PO adducts, *Rosa roxburghii* extract, *Achillea millefolium* extract, *Melilotus officinalis* extract, and the like.

The oily components can be exemplified by liquid oils, solid fats, waxes, hydrocarbons, higher fatty acids, higher alcohols, synthetic ester oils, silicone oils, and the like. It should be noted that in this specification, oil fractions and components soluble in oil fractions are also included under the term "oily components".

The liquid oils can be exemplified by avocado oil, camellia oil, turtle oil, macadamia oil, corn oil, mink oil, olive oil, rapeseed oil, yolk oil, sesame oil, peach kernel oil (almond oil), wheat germ oil, camellia flower oil, castor oil, flaxseed oil, safflower oil, cottonseed oil, perilla seed oil, soybean oil, peanut oil, tea seed oil, ginkgo nut oil, rice bran oil, Chinese tung oil, Japanese tung oil, jojoba oil, germ oil, triglycerides, and the like.

The solid fats can be exemplified by cocoa butter, coconut oil, horse oil, hydrogenated coconut oil, palm oil, beef tallow, mutton tallow, hydrogenated beef tallow, palm kernel oil, pork lard, beef bone fat (oil), wood kernel oil, hydrogenated oil, neat's foot oil, wood wax, hydrogenated castor oil, and the like.

The waxes can be exemplified by beeswax, candelilla wax, cotton wax, carnauba wax, bayberry wax, insect wax (white wax), spermaceti wax, lignite wax, rice bran wax, lanolin, kapok wax, acetylated lanolin, liquid lanolin, sugarcane wax, isopropyl lanolate, hexyl laurate, reduced lanolin, jojoba wax, hard lanolin, shellac wax, POE lanolin alcohol ether, POE lanolin alcohol acetate, POE cholesterol ether, lanolin fatty acid polyethylene glycol ester, POE hydrogenated lanolin alcohol ether, and the like.

The hydrocarbon oils can be exemplified by liquid paraffin, ceresin wax, squalane, pristane, paraffin wax, microcrystalline wax, squalene, petrolatum, microcrystalline wax, and the like.

The hydrocarbon oils can be exemplified by liquid paraffin, ceresin, squalane, pristane, paraffin, pure ceresin, squalene, petrolatum, microcrystalline wax, and the like.

The higher fatty acids can be exemplified by lauric acid, myristic acid, palmitic acid, stearic acid, behenic acid, oleic acid, undecylenic acid, tall oil fatty acids, isostearic acid, linoleic acid, linolenic acid, eicosapentaenoic acid (EPA), docosahexaenoic acid (DHA), and the like.

The higher alcohols can be exemplified by straight-chain alcohols (e.g., lauryl alcohol, cetyl alcohol, stearyl alcohol, behenyl alcohol, myristyl alcohol, oleyl alcohol, cetearyl alcohol, etc.); branched-chain alcohols (e.g., monostearyl glycerol ether (batyl alcohol), 2-decyltetradecanol, lanolin alcohol, cholesterol, phytosterols, hexyl dodecanol, isostearyl alcohol, octyl dodecanol, etc.), and the like.

The synthetic ester oils can be exemplified by hydrogenated polydecene, isopropyl myristate, cetyl octanoate, octyldodecyl myristate, isopropyl palmitate, butyl stearate, hexyl laurate, myristyl myristate, decyl oleate, hexyl decyl dimethyloctanoate, cetyl lactate, myristyl lactate, lanolin acetate, isocetyl stearate, isostearyl isostearate, cholesterol 12-hydroxystearate, di-2-ethylhexyl adipate, dipentaerythritol fatty acid ester, mono-isostearoyl N-alkyl ethylene glycol ester, neopentyl glycol dicaprate, diisostearoyl malate, di-2-heptylundecyl glycerol ester, tri-2-ethylhexyl trimethylolpropane ester, triisostearoyl trimethylolpropane ester, pentaerythritol tetra-2-ethylhexanoate, tri-2-ethylhexyl glycerol ester, trioctanoin, triisopalmitin, trimethylolpropane triisostearate, cetyl ethylhexanoate, 2-ethylhexyl palmitate, trimyristin, tri-2-heptylundecyl glycerol ester, methyl ricinoleate, oleoyl oleate, acetylated glycerides, palmitic acid 2-heptylundecyl ester, diisobutyl adipate, N-lauroyl-L-glutamic acid 2-octyldodecyl ester, di-2-heptylundecyl adipate, ethyl laurate, di-2-ethylhexyl sebacate, myristyl 2-hexyldecyl ester, palmitic acid 2-hexyldecyl ester, adipic acid 2-hexyldecyl ester, diisopropyl sebacate, 2-ethylhexyl succinate, triethyl citrate, and the like.

The silicone oils can be exemplified by polydimethylsiloxane, methyl hydrogen polysiloxane, methylphenyl polysiloxane, stearoyloxy methyl polysiloxane, polyether-modified organic polysiloxane, fluoroalkyl·polyoxyalkylene co-modified organic polysiloxane, alkyl-modified organic polysiloxane, end-modified organic polysiloxane, fluoro-modified organic polysiloxane, amino-modified organic polysiloxane, PEG-10 polydimethylsiloxane, silicone gel, acrylic polysiloxane, trimethylsiloxysilicate, silicone compounds such as organic silicone RTV rubber, and the like.

As the oil component used in combination with hydrogenated polyisobutene, it is preferable to select an oil that has poor compatibility with hydrogenated polyisobutene and is highly volatile. In this way, during application, the volatile oil will evaporate, and due to the poor compatibility, the hydrogenated polyisobutene will remain on the skin to form a film, providing a tightening effect.

The anionic surfactants can be exemplified by fatty acid soaps (e.g., sodium laurate, sodium palmitate, etc.); higher alkyl sulfates (e.g., sodium lauryl sulfate, potassium lauryl sulfate, etc.); alkyl ether sulfates (e.g., triethanolamine POE-lauryl sulfate, sodium POE-lauryl sulfate, etc.); N-acylsarcosinic acids (e.g., sodium lauroylsarcosinate, etc.); higher fatty acid amide sulfates (e.g., sodium N-myristoyl-N-methyltaurate, sodium cocoyl methyltaurate, sodium lauroyl methyltaurate, etc.); phosphates (sodium POE-oleyl ether phosphate, POE-stearyl ether phosphoric acid, etc.); sulfosuccinates (e.g., sodium di-2-ethylhexylsulfosuccinate, sodium monolauroyl monoethanolamide polyoxyethylenesulfosuccinate, sodium lauryl polypropyleneglycol sulfosuccinate, etc.); alkylbenzenesulfonates (e.g., sodium linear dodecylbenzenesulfonate, triethanolamine linear dodecylbenzenesulfonate, linear dodecylbenzenesulfonic acid, etc.); higher fatty acid ester sulfates (e.g., sodium hydrogenated cocoglyceride sulfate, etc.); N-acylglutamates (e.g., monosodium N-lauroylglutamate, disodium N-stearoylglutamate, monosodium N-myristoyl-L-glutamate, etc.); sulfated oils (e.g., Turkey red oil, etc.); POE-alkyl ether carboxylic acids, POE-alkyl allyl ether carboxylates, $\alpha$-olefmsulfonates, higher fatty acid ester sulfonates, secondary alcohol sulfates, higher fatty acid alkylolamide sulfate, sodium lauroyl monoethanolamide succinate, ditriethanolamine N-palmitoylaspartate, sodium caseinate, and the like.

The cationic surfactants can be exemplified by alkyl trimethyl ammonium salts (e.g., stearyl trimethyl ammonium chloride, lauryl trimethyl ammonium chloride, etc.); alkyl pyridinium salts (e.g., cetylpyridinium chloride, etc.); distearyl dimethyl ammonium chloride ; poly(N,N"-dimethyl-3,5-methylenepiperidinium) chloride; alkyl quaternary ammonium salts; alkyl dimethyl benzyl ammonium salts; alkylisoquinolinium salts; dialkylmorphonium salts; POE-alkylamines; alkylamine salts; polyamine fatty acid derivatives; amyl alcohol fatty acid derivatives; benzalkonium chloride; benzethonium chloride, and the like.

The amphoteric surfactants can be exemplified by imidazoline series amphoteric surfactants (e.g., 2-undecyl-N,N,N-(hydroxyethylcarboxymethyl)-2-imidazoline sodium, 2-cocoyl-2-imidazolinium hydroxide-1-carboxyethyloxy disodium salt, etc.); betaine series surfactants (e.g., 2-heptadecyl-N-carboxymethyl-N-hydroxyethylimidazolinium betaine, lauryldimethylaminoacetic acid betaine, alkylbetaine, amidebetaine, sulfobetaine, etc.), and the like.

The lipophilic nonionic surfactants can be exemplified by sorbitan fatty acid esters (e.g., sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2-ethylhexanoate, diglycerol sorbitan tetra-2-ethylhexanoate, etc.); glycerin polyglycerin fatty acids (e.g., mono-cottonseed fatty acid glycerin, monoerucic acid glycerol, sesquioleic acid glycerol, glyceryl monostearate, $\alpha,\alpha'$-glyceryl oleate pyroglutamate, glyceryl monostearate malate, etc.); propylene glycol fatty acid esters (e.g., monostearic acid propylene glycol, etc.); hydrogenated castor oil derivatives; glycerin alkyl ethers; PEG-10 polydimethylsiloxane, glyceryl stearate, PPG-13-decyltetradecanol polyether-24, PEG-5 glyceryl stearate, and the like.

The hydrophilic nonionic surfactants can be exemplified by POB-sorbitan fatty acid esters (e.g., POE-sorbitan monooleate, POE-sorbitan monostearate, POE-sorbitan monooleate, POE-sorbitan tetraoleate, etc.); POE-sorbitol fatty acid esters (e.g., POE-sorbitol monolaurate, POE-sorbitol monooleate, POE-sorbitol pentaoleate, and POE-sorbitol monostearate, etc.); POE-glycerol fatty acid esters (e.g., POE-glycerol monostearate, POE-glycerol monoisostearate,

POE-glycerol triisostearate, etc.); POE-fatty acid esters (e.g., POE-monooleate, POE-distearate, POE-monodioleate, ethylene glycol distearate, etc.); POE-alkyl ethers (e.g., POE-lauryl ether, POE-oleyl ether, POE-stearyl ether, POE-behenyl ether, POE-2-octyldodecyl ether, POE-cholestanol ether, etc.); Pluronic type surfactants (e.g., Pluronic, etc.); POE•POP-alkyl ethers (e.g., POE·POP-cetyl ether, POE•POP-2-decyltetradecyl ether, POE·POP-monobutyl ether, POE•POP-hydrogenated lanolin, POE•POP-glyceryl ether, etc.); tetra-POE•tetra-POP-ethylenediamine condensates (e.g., Tetronic, etc.); POE-castor oil hydrogenated castor oil derivatives (e.g., POE-castor oil, POE-hydrogenated castor oil, POE-hydrogenated castor oil monoisostearate, POE-hydrogenated castor oil triisostearate, POE-hydrogenated castor oil monopyroglutamic monoisostearic diester, POE-hydrogenated castor oil maleate, etc.); POE-beeswax•lanolin derivatives (e.g., POE-sorbitol beeswax, etc.); alkanol amides (e.g., palm oil fatty acid diethanol amide, laurate monoethanolamide, fatty acid isopropanol amide, etc.); POE-propylene glycol fatty acid esters; POE-alkylamines; POE-fatty acid amides; sucrose fatty acid esters; alkyl ethoxydimethylamine oxides; trioleyl phosphate; oleth-10; PEG-100 stearate; methoxy PEG/PPG-25/4 dimethicone; polysorbate 60; PEG-40 stearate; sucrose stearate. Examples of natural water-soluble polymers include plant-based polymers (e.g., gum arabic, gum tragacanth, galactan, guar gum, carob gum, karaya gum, carrageenan, pectin, agar, quince seed, algae colloids (brown algae extract), starches (rice, corn, potato, and wheat), and glycyrrhizic acid); microorganism-based polymers (e.g., xanthan gum, dextran, succinoglucan, pullulan, etc.); animal-derived polymers (e.g., collagen, casein, albumin, gelatin, etc.), and the like.

The semi-synthetic water-soluble polymers can be exemplified by starch-based polymers (e.g., carboxymethyl starch, methylhydroxypropyl starch, etc.); cellulose-based polymer compounds (methyl cellulose, ethyl cellulose, methyl hydroxypropyl cellulose, hydroxyethyl cellulose, sodium cellulose sulfate, hydroxypropyl cellulose, carboxymethyl cellulose, sodium carboxymethyl cellulose, crystalline cellulose, cellulose powder, etc.); alginic acid polymers (e.g., sodium alginate, the propylene glycol ester of alginic acid, etc.); PEG-240/HDI copolymer bis-decyltetradeceth-20, acrylates/C10-30 alkyl acrylate crosspolymer, and the like.

The thickeners can be exemplified by carbomer, acrylates/C10-30 alkyl acrylate crosspolymer, sodium acrylate/sodium acryloyldimethyl taurate copolymer, acrylates/cetyl ethoxy (20) itaconate copolymer, acrylates/cetyl ethoxy (20) methacrylate copolymer, acrylates/tetradecyl ethoxy (25) acrylate copolymer, acrylates/octadecyl ethoxy (20) itaconate copolymer, acrylates/octadecyl ethoxy (20) methacrylate copolymer, acrylates/octadecyl ethoxy (50) acrylate copolymer, acrylates/VA crosspolymer, PAA (polyacrylic acid), sodium acrylate/ethylene isodecanol crosspolymer, carbomer (polyacrylic acid) and its sodium salt and other polyacrylic acid thickeners.

The preservatives can be exemplified by phenoxyethanol, benzyl alcohol, methylparaben, phenoxypropanol, and other aromatic preservatives.

The ultraviolet absorbers can be exemplified by benzoic acid-based ultraviolet absorbers (e.g., paraaminobenzoic acid (hereinafter, abbreviated as PABA), PABA monoglycerin ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc.); anthranilic acid series ultraviolet absorbers (e.g., homomenthyl-N-acetyl anthranilate, etc.); salicylic acid-based ultraviolet absorbers (e.g., amyl salicylate, menthyl salicylate, homomenthyl salicylate, octyl salicylate, phenyl salicylate, benzyl salicylate, p-isopropanol phenyl salicylate, etc.); cinnamic acid-based ultraviolet absorbers (e.g., octyl cinnamate, ethyl-4-isopropyl cinnamate, methyl-2,5-diisopropyl cinnamate, ethyl-2,4-diisopropyl cinnamate, methyl-2,4-diisopropyl cinnamate, propyl-p-methoxy cinnamate, isopropyl-p-methoxy cinnamate, isoamyl-p- methoxy cinnamate, octyl-p-methoxy cinnamate (2-ethylhexyl-p-methoxy cinnamate), 2-ethoxyethyl-p-methoxy cinnamate, cyclohexyl-p-methoxy cinnamate, ethyl-α-cyano-β-phenyl cinnamate, 2-ethylhexyl-α-cyano-β-phenyl cinnamate, glyceryl mono-2-ethylhexanoyl-diparamethoxy cinnamate, etc.); benzophenone-based ultraviolet absorbers (e.g., 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenylbenzophenone-2-carboxylate, 2-hydroxy-4-n-octoxybenzophenone, 4-hydroxy-3-carboxybenzophenone, etc.); 3-(4'-methylbenzylidene)-d,l-camphor; 3-benzylidene-d,l-camphor; 2-phenyl-5-methylbenzoxazole; 2,2'-hydroxy-5-methylphenylbenzotriazole; 2-(2'-hydroxy-5'-t-octylphenyl)benzotriazole; 2-(2'-hydroxy-5'-methylphenyl)benzotriazole; dibenzalazine; dianisoylmethane; 4-methoxy-4'-t-butyldibenzoylmethane; 5-(3,3-dimethyl-2-norbonylidene)-3-pentan-2-one; dimorpholino pyridazinone; 2-ethylhexyl-2-cyano-3,3-diphenylacrylate; 2,4-bis-{[4-(2-ethylhexyloxy)-2-hydroxy]-phenyl}-6-(4-methoxyphenyl)-(1,3,5)-triazine, and the like.

The metal ion sequestrants can be exemplified by 1-hydroxyethane-1,1-diphosphonic acid, tetrasodium 1-hydroxyethane-1,1-diphosphonate, disodium edetate (EDTA2 sodium), trisodium edetate (EDTA3 sodium), tetrasodium edetate, sodium citrate, sodium polyphosphate, sodium meta-phosphate, gluconic acid, phosphoric acid, citric acid, ascorbic acid, succinic acid, edetic acid, trisodium ethylenediaminehydroxyethyltriacetate, and the like.

The amino acids can be exemplified by neutral amino acids (e.g., threonine, cysteine, etc.); basic amino acids (e.g., hydroxylysine, etc.). Besides, the amino acid derivatives can be exemplified by sodium acylsarcosinate (sodium lauroylsarcosinate), acylglutamate salts, sodium acyl-β-alanine, glutathione, pyrrolidonecarboxylic acid, and the like.

The organic amines can be exemplified by monoethanolamine, diethanolamine, triethanolamine, morpholine,

triisopropanolamine, 2-amino-2-methyl-1,3-propanediol, 2-amino-2-methyl-1-propanol, and the like.

The polymer emulsions can be exemplified by acrylic resin emulsions, polyethyl acrylate emulsions, acrylic resin liquids, polyalkyl acrylate emulsions, polyvinyl acetate resin emulsions, natural rubber latexes, etc.

The pH modifiers can be exemplified by buffers, for example, lactic acid-sodium lactate, citric acid-sodium citrate, succinic acid-sodium succinate, and the like.

The neutralizing agents can be exemplified by potassium hydroxide, sodium hydroxide, aminomethylpropanol, arginine, and the like.

The vitamins can be exemplified by vitamins A, B1, B2, B6, C, E and their derivatives, pantothenic acid and derivatives thereof, biotin, and the like.

The antioxidants can be exemplified by tocopherols, dibutylhydroxytoluene, butylhydroxyanisole, gallic acid esters, and the like.

The antioxidant aids can be exemplified by phosphoric acid, citric acid, ascorbic acid, maleic acid, malonic acid, succinic acid, fumaric acid, cephalin, hexametaphosphate, phytic acid, ethylenediaminetetraacetic acid, and the like.

## Test Examples

### Test Example 1. Effect of α-Ionone in Promoting the Proliferation of HaCaT Cells

The effect of α-ionone in promoting the proliferation of HaCaT cells was confirmed using the CCK8 (Cell Counting Kit-8) assay. HaCaT cells, which are immortalized human keratinocytes derived from normal adult skin, possess biological characteristics similar to those of primary keratinocytes and retain the ability to differentiate in the epidermis, and are commonly used as an *in vitro* alternative model for keratinocyte-related epidermal biological studies.

HaCaT cells were seeded at a density of $5\times10^3$ cells per well in a 96-well cell culture plate and cultured in a cell incubator for 24 h. Subsequently, different concentrations of α-ionone (1-400 μM) were added, and the cells were treated for either 24 h (Figure 1, upper panel) or 48 h (Figure 1, lower panel). After the culture, 10 μl of CCK-8 solution was added to each well, and the culture plate was incubated in the incubator for 1 h. After incubation, the absorbance OD value at 450 nm was measured for each well using a microplate reader. The proliferation rate of HaCaT cells was calculated based on the percentage of OD values in each experimental group relative to the control group. The results are shown in Figure 1. The data presented in the figure represent the Mean ± SD derived from three replicate experiments, with $^{**}p < 0.01$ vs. blank control group.

As can be seen from the results in Figure 1, α-ionone effectively promotes the proliferation of HaCaT cells within the dose range of 1-200 μM, and it exhibits the strongest cell proliferation effect at a dose of 50 μM. At a dose higher than 100 μM, its proliferation effect is not further enhanced, but weakened (Figure 1). Therefore, α-ionone can directly act on HaCaT cells to promote proliferation, with an effective dose range of 1-200 μM.

### Test Example 2. Effect of α-Ionone in Promoting the Migration of HaCaT Cells

The effect of α-ionone in promoting the migration of HaCaT cells was confirmed using the scratch assay.

HaCaT cells were seeded at a density of $2\times10^5$ cells per well in a 6-well cell culture plate and cultured in a cell incubator until the cells reached 90% confluence. The culture medium was then aspirated, and a sterile 20μl pipette tip was used to create a "scratch" by drawing a straight line perpendicular to the cell monolayer. After washing with PBS, 2 mL of complete culture medium containing different concentrations of α-ionone (1, 10, 25, 50 μM) was added, followed by further culture for 24 h. The cells were observed and photographed under an inverted microscope at different time points post-scratching (0, 12, and 24 h). The scratch area was calculated using the Image J software, and the cell migration rate was calculated using the following formula:

Cell migration rate (%) = (Initial scratch area - Scratch area after culture) / Initial scratch area × 100%

The results are shown in Figure 2. The data in the figure represent the Mean ± SD (n=4), with $^{*}p<0.05$ vs. vehicle control group.

As can be seen from the results in Figure 2, α-ionone promotes the migration of HaCaT cells in a dose-dependent manner within the dose range of 10-50 μM. Although a low dose of 1 μM induced a certain degree of increase in the cell migration rate, this effect did not reach statistical significance compared to the blank control group, suggesting that α-ionone is largely ineffective at doses below 1 μM. Compared to the 25 μM dose group, the 50 μM dose group of α-ionone did not show a significant increase in cell migration rate, and it even slightly decreased at 24 hours, suggesting that further increasing the dose of α-ionone does not further enhance its cell migration-promoting effect (Figure 2). Therefore, α-ionone can promote the migration of HaCaT cells, with an effective dose range of 10-50 μM.

**Test Example 3: α-Ionone Effectively Attenuates the Inhibitory Effect of Norepinephrine (NE) on the Migration of HaCaT Cells**

It is known that the stress hormone norepinephrine (NE) inhibits the migration of keratinocytes, thereby delaying skin wound healing. To detect the impact of α-ionone on NE-induced inhibition of HaCaT cell migration, 10 μM NE or NE (10 μM) + α-ionone (25, 50 μM) was added to the confluent monolayer of HaCaT cells after scratching, and the cells were cultured for 24 h. The cells were observed and photographed under an inverted microscope at different time points post-scratching (0, 12, and 24 h). The scratch area was calculated using the Image J software, and the cell migration rate was calculated. The results are shown in Figure 3. The data in the figure represent the Mean ± SD (n=4), with *$p$<0.05 vs. blank control group, $^{\#}p$<0.05 vs. NE alone group.

As shown in Figure 3, treatment with NE (10 μM) alone significantly inhibits HaCaT cell migration. In contrast, treatment with the experimental group of NE + α-ionone (25, 50 μM) significantly increases the cell migration rate compared to the NE alone group, indicating that α-ionone effectively attenuates NE-induced suppression of cell migration.

**Test Example 4: Impact of α-Ionone on HAS-2 Expression in HaCaT Cells**

The impact of α-ionone alone on the expression of the HAS-2 gene was confirmed in normally cultured HaCaT cells.

HaCaT cells were seeded at a density of $2\times10^5$ cells per well in a 6-well cell culture plate and cultured in a cell incubator for 24 h. Subsequently, different concentrations of α-ionone (1, 10, 25, 50, 100 μM) were added and the cells were treated for 9 h. The mRNA expression levels of HAS-2 in the cells were determined by quantitative real-time PCR (qPCR). The results are shown in Figure 4. The data in the figure represent the Mean ± SD from three replicate experiments, with **$p$<0.01.

As can be seen from the results in Figure 4, α-ionone increases the expression level of the HAS-2 gene in a dose-dependent manner within the dose range of 10-50 μM. At a higher dose of 100 μM, the pharmacological activity does not further enhance but rather weakens. Therefore, α-ionone has a promoting effect on the expression of HAS-2 in HaCaT cells, with an effective dose range of 10-50 μM.

**Test Example 5: Effect of α-Ionone on the Synthesis of HA in HaCaT Cells**

In this test, the Inventors quantitatively measured the levels of HA in the culture supernatant of HaCaT cells after stimulation with α-ionone for 24 h.

HaCaT cells were seeded at a density of $2\times10^5$ cells per well in a 6-well cell culture plate and cultured in a cell incubator for 24 hours. Subsequently, different concentrations of α-ionone (25, 50 μM) were added and the cells were treated for 24 h. The cell culture supernatant was collected and the concentration of HA in the cell culture supernatant was determined by ELISA. The results are shown in Figure 5. The data in the figure represent the Mean ± SD from three replicate experiments, with **$p$ <0.01.

As can be seen from the results in Figure 5, treatment with α-ionone (25, 50 μM) for 24 h significantly increases the levels of HA in the culture supernatant of HaCaT cells, indicating that α-ionone promotes the synthesis of HA by upregulating the expression of hyaluronic acid synthase HAS-2. Therefore, these results further confirm the pharmacological activity of α-ionone in inducing the expression of hyaluronic acid synthase.

**Test Example 6. Impact of α-Ionone on Dexamethasone (DEX)-Induced Inhibition of HAS-2 Expression**

Glucocorticoids reduce the synthesis of HA by inhibiting the expression of HAS-2 in keratinocytes, which is one of the main pathological mechanisms underlying skin atrophy and skin barrier dysfunction caused by glucocorticoids and psychological stress. DEX is a synthetic, potent glucocorticoid drug with greater activity and stability than endogenous glucocorticoids, and is therefore commonly used as a tool for studying the effects of glucocorticoids. HaCaT cells stimulated by DEX are often used as a skin stress cell model. This test investigates the impact of α-ionone on the inhibition of HAS-2 expression by DEX in a DEX-induced skin stress cell model.

HaCaT cells were seeded at a density of $2\times10^5$ cells per well in a 6-well cell culture plate and cultured in a cell incubator for 24 h. Different concentrations of α-ionone (1, 10, 25, 50, 100 μM) were added and the cells were treated for 3 h. Subsequently, 1 μM DEX was added and the cells were treated for 6 h. The mRNA expression level of HAS-2 in the cells was determined by qPCR. The results are shown in Figure 6. The data in the figure represent the Mean ± SD from three replicate experiments, with *$p$<0.05, **$p$<0.01.

As can be seen from the results in Figure 6, DEX (1 μM) significantly inhibits the expression of HAS-2 in HaCaT cells. α-Ionone in the dose range of 10-50 μM can effectively alleviate the inhibitory effect of DEX on HAS-2 expression, indicating that it can enhance the expression level of HAS-2 in keratinocytes under the influence of stress hormones.

Therefore, α-ionone has an antagonistic effect on the DEX-induced inhibition of HAS-2 expression, with an effective dose range of 10-50 μM.

**Test Example 7. Effect of α-Ionone in Promoting the HBD-2 Expression in HaCaT Cells**

The impact of α-ionone alone on the expression of the HBD-2 gene was confirmed in normally cultured HaCaT cells.

HaCaT cells were seeded at a density of $2\times10^5$ cells per well in a 6-well cell culture plate and cultured in a cell incubator for 24 h. Different concentrations of α-ionone (1, 10, 25, 50, 100 μM) were added and the cells were treated for 9 h. The mRNA expression level of HBD-2 in the cells was determined by qPCR. The results are shown in Figure 7. The data in the figure represent the Mean ± SD from three replicate experiments, with $^{**}p<0.01$.

As can be seen from the results in Figure 7, α-ionone in the dose range of 10-50 μM can effectively enhance the expression level of the HBD-2 gene. These results confirm the pharmacological activity of α-ionone in inducing the expression of HBD-2.

**Test Example 8. Impact of α-Ionone on the HBD-2 Secretion by HaCaT Cells**

In this test, the Inventors quantitatively measured the levels of HBD-2 in the culture supernatant of HaCaT cells after stimulation with α-ionone for 24 h.

HaCaT cells were seeded at a density of $2\times10^5$ cells per well in a 6-well cell culture plate and cultured in a cell incubator for 24 hours. Subsequently, different concentrations of α-ionone (25, 50 μM) were added and the cells were treated for 24 h. The cell culture supernatant was collected and the concentration of HBD-2 in the cell culture supernatant was determined by ELISA. The results are shown in Figure 8. The data in the figure represent the Mean ± SD from three replicate experiments, with $^{**}p<0.05$.

As can be seen from the results in Figure 8, treatment with α-ionone (25, 50 μM) for 24 hours significantly increases the levels of HBD-2 in the culture supernatant of HaCaT cells, indicating that α-ionone promotes the synthesis and secretion of HBD-2 by enhancing the expression of HBD-2. Therefore, these results further confirm the pharmacological activity of α-ionone in inducing the expression of HBD-2.

**Test Example 9. Impact of α-Ionone on Dexamethasone (DEX)-Induced Inhibition of HBD-2 Expression in HaCaT Cells**

The inhibition of HBD expression in keratinocytes by glucocorticoids is one of the key pathological mechanisms underlying the increased susceptibility to microbial infections and the impairment of epidermal barrier function caused by glucocorticoid use and psychological stress. This test further demonstrates the impact of α-ionone on DEX-induced inhibition of HBD-2 expression in a DEX-induced skin stress cell model.

HaCaT cells were seeded at a density of $2\times10^5$ cells per well in a 6-well cell culture plate and cultured in a cell incubator for 24 hours. Different concentrations of α-ionone (1, 10, 25, 50, 100 μM) were added and the cells were treated for 3 h. Subsequently, 1 μM DEX was added and the cells were treated for 6 h. The mRNA expression level of HBD-2 in the cells was determined by qPCR. The results are shown in Figure 9. The data in the figure represent the Mean ± SD from three replicate experiments, with $^{**}p<0.01$.

As can be seen from the results in Figure 9, DEX (1 μM) can significantly inhibit the expression of HBD-2 in HaCaT cells. α-Ionone in the dose range of 10-50 μM can effectively alleviate the inhibitory effect of DEX on HBD-2 expression and enhance the expression level of HBD-2 in keratinocytes under the influence of stress hormones. These results indicate that α-ionone has an antagonistic effect on the DEX-induced inhibition of HBD-2 expression, with an effective dose range of 10-50 μM.

**Examples**

**Example 1. Toner**

According to the formulation listed below, a toner containing α-ionone as an active ingredient for skin barrier repair was prepared using conventional cosmetic manufacturing processes.

| Ingredient | Content (mss%) |
|---|---|
| Water | q.s. to 100% |
| Dipropylene glycol | 5 |

(continued)

| Ingredient | Content (mss%) |
|---|---|
| PPG-13-Decyltetradeceth-24 | 1 |
| Phenoxyethanol | 0.6 |
| EDTA Disodium | 0.02 |
| α-Ionone | 0.02 |

**Example 2. Toner**

According to the formulation listed below, a toner containing α-ionone as an active ingredient for skin barrier repair was prepared using conventional cosmetic manufacturing processes.

| Ingredient | Content (mss%) |
|---|---|
| Water | q.s. to 100% |
| Dipropylene glycol | 5 |
| PPG-13-Decyltetradeceth-24 | 1 |
| Phenoxyethanol | 0.6 |
| EDTA Disodium | 0.02 |
| α-Ionone | 0.1 |

**Example 3. Emulsion**

According to the formulation listed below, an emulsion containing α-ionone as an active ingredient for skin barrier repair was prepared using conventional cosmetic manufacturing processes.

| Ingredient | Content (mss%) |
|---|---|
| Water | q.s. to 100% |
| Butanediol | 5 |
| Carbomer | 0.12 |
| Potassium hydroxide | 0.08 |
| Behenic acid | 0.3 |
| Stearic acid | 0.2 |
| Isostearic acid | 0.3 |
| Glyceryl stearate | 1.2 |
| PEG-5 Glyceryl stearate | 1.2 |
| Dimethicone | 1 |
| Cetyl ethylhexanoate | 3 |
| Methylparaben | 0.2 |
| α-Ionone | 0.02 |

**Example 4. Emulsion**

According to the formulation listed below, an emulsion containing α-ionone as an active ingredient for skin barrier repair was prepared using conventional cosmetic manufacturing processes.

| Ingredient Content (mss%) | |
|---|---|
| Water | q.s. to 100% |
| Butanediol | 5 |
| Carbomer | 0.12 |
| Potassium hydroxide | |
| Behenic acid | |
| Stearic acid | 0.2 |

(continued)

| Ingredient | Content (mss%) |
|---|---|
| Isostearic acid | |
| Glyceryl stearate | |
| PEG-5 Glyceryl stearate | |
| Dimethicone | |
| Cetyl ethylhexanoate | |
| Methylparaben | |
| α-Ionone | 0.1 |

**Example 5. Cream**

According to the formulation listed below, a cream containing α-ionone as an active ingredient for skin barrier repair was prepared using conventional cosmetic manufacturing processes.

| Ingredient | Content (mss%) |
|---|---|
| Water | q.s. to 100% |
| Ethanol | 5 |
| Dipropylene glycol | 5 |
| Dimethicone | 3 |
| PEG-240/HDI copolymer bis-decyltetrade ceth-20 | 1.2 |
| Carbomer | 0.3 |
| Acrylates/C10-30 alkyl acrylate crosspoly mer | 0.03 |
| Potassium hydroxide | 0.1 |
| Phenoxyethanol | 0.5 |
| α-Ionone | 0.02 |

**Example 6. Cream**

According to the formulation listed below, a cream containing α-ionone as an active ingredient for skin barrier repair was prepared using conventional cosmetic manufacturing processes.

| Ingredient | Content (mss%) |
|---|---|
| Water | q.s. to 100% |
| Ethanol | 5 |
| Dipropylene glycol | 5 |
| Dimethicone | 3 |
| PEG-240/HDI copolymer bis-decyltetrade ether | 1.2 |
| Carbomer | 0.3 |
| Acrylates/C10-30 alkyl acrylate crosspoly mer | 0.03 |
| Potassium hydroxide | 0.1 |
| Phenoxyethanol | 0.5 |
| α-Ionone | 0.1 |

**Example 7. Hydrogel**

According to the formulation listed below, a hydrogel containing α-ionone as an active ingredient for skin barrier repair was prepared.

An appropriate amount of deionized water was heated to 85°C in a water bath. Carbomer U20 powder was slowly added to the water and stirred until dissolved uniformly, followed by neutralization with 10% arginine. p-Hydroxyacetophenone and 1,2-hexanediol were weighed and dissolved in an appropriate amount of deionized water with gentle heating, then added to the Carbomer U20 gel and stirred until uniform to obtain a hydrogel matrix. When the temperature of

the hydrogel matrix dropped below 40°C, α-ionone was added and stirred until uniform to obtain an α-ionone hydrogel.

| Ingredient | Content (g) |
|---|---|
| α-Ionone | 0.1 |
| Carbomer U20 | 0.25 |
| Arginine (10%) | 2.5 |
| p-Hydroxyacetophenone | 0.4 |
| 1,2-Hexanediol | 0.4 |
| Deionized water | q.s. to 100 g |

**Example 8. Hydrogel**

According to the formulation listed below, a hydrogel containing α-ionone as an active ingredient for skin barrier repair was prepared using the manufacturing method described in Example 7.

| Ingredient | Content (g) |
|---|---|
| α-Ionone | 0.1 |
| Carbomer U20 | 0.25 |
| Arginine (10%) | 2.5 |
| p-Hydroxyacetophenone | 0.4 |
| 1,2-Hexanediol | 0.4 |
| Deionized water | q.s. to 100 g |

**Efficacy Test Example**

**1. Test Principle**

In human clinical trials, the primary method involves subjective evaluation and objective assessment of skin parameters to analyze the improvement in skin condition before and after the use of test samples. The results are more intuitive and are suitable for evaluating the efficacy of finished or semi-finished cosmetic products.

In this trial, a group of healthy individuals was recruited to form the subject population. A skin barrier damage model was created on the flexor side of the arm using tape stripping. The changes in physiological parameters such as the moisture content of the stratum corneum and the rate of transepidermal water loss (TEWL) were measured before and after the application of the cosmetic product on the flexor side of the arm, thereby evaluating the efficacy of cosmetics in promoting skin barrier repair.

2. Materials and Instruments

2.1 Test samples:

| No. | Sample Name | Notes |
|---|---|---|
| **1** | **Blank hydrogel matrix** | **Blank hydrogel without α-ionone** |
| **2** | **0.1% α-ionone hydrogel (Example 7)** | **Hydrogel containing 0.1% α-ionone** |
| **3** | **1% α-ionone hydrogel (Example 8)** | **Hydrogel containing 1% α-ionone** |

Method of applying the test sample: The test product was applied to the tape-stripped area on the flexor side of the arm once daily at a dosage of approximately 2 mg/$cm^2$. An area subjected to tape stripping but without application of the product was set as the model control.

### 2.2 Instruments

A device for measuring stratum corneum moisture content (Corneometer CM 825, C&K, Germany); a device for measuring transepidermal water loss (Vapometer, Delfin, Finland).

## 3. Test Methods

### 3.1 Subjects

According to the Declaration of Helsinki, the selection of subjects adheres to the medical and ethical standards for human testing. All subjects must be tested voluntarily and sign informed consent before the test. Before the subject signs the informed consent, the tester needs to inform the subject of the purpose of the test, possible benefits, potential risks and problems, and related rights and obligations.

#### 3.1.1 Number of Subjects

Target number of subjects: 32 individuals

#### 3.1.2 Inclusion Criteria:

Aged between 18 and 65 years old;
Having healthy and evenly pigmented skin on the flexor side of the arm, free from scars or other conditions that may affect the test;
Being able to return for follow-up visits and undergo skin tests as required.

#### 3.1.3 Exclusion Criteria:

Pregnant or breastfeeding;
Individuals with a history of allergies to skincare or personal care products, or are allergic to any ingredient in the test product and its accompanying samples;
Individuals having severe systemic diseases and other skin diseases, such as lupus erythematosus, psoriasis, etc.;
Individuals having received beauty therapy in the test area;
Individuals who are currently participating in or have participated in other clinical trials prior to the start of this study.

#### 3.1.4 Withdrawal (dropout) criteria:

Subjects lost to follow-up or actively requested to withdraw;
Poor compliance, such as failure to use the test products as instructed, or failure to attend follow-up visits as required;
Use of other cosmetics similar to the test products during the study;
Development of any new disease that directly affects the clinical status assessment, including skin diseases, during the study period;
Development of a serious illness during the study period;
Pregnancy during the study period;
Inability to tolerate the study procedures;
Occurrence of a serious adverse event (SAE) during the study period.

### 3.2 Test Indicators

#### 3.2.1 Stratum Corneum Moisture Content

The moisture content of the stratum corneum was measured using the Corneometer CM 825 probe of the MPA 580 Multiprobe Adaptor System (CK, Germany). This probe employs the capacitive method to measure the moisture

content of the skin surface. The dielectric constant of water in the skin surface is much higher than that of other substances. Changes in the dielectric constant of the skin surface are primarily due to changes in the moisture content of the skin surface. By measuring the dielectric constant of the skin surface, the moisture content of the skin surface can be analyzed. The measurement value is a relative value, expressed in C. U. (Corneometer Units). A higher value indicates a higher moisture content in the skin surface.

### 3.2.2 Transepidermal Water Loss (TEWL)

Transepidermal water loss (TEWL) was measured using the Vapometer (Delfin, Finland). The device has a circular opening at the top, which, upon contact with the skin, forms a sealed chamber. A high-precision humidity sensor within the chamber records changes in the humidity of the air inside. By measuring the rate of change in air humidity over a specific time period after contact with the skin, the rate of water loss through the skin can be calculated. The measurement value is expressed in units of g/($m^2$•h). A lower value indicates a lower rate of water loss from the skin surface, which suggests better skin barrier function.

### 3.3 Measurement Sites

Four 3cm*3cm areas were selected on the flexor side of the arm as test sites, with at least 1cm spacing between each area. Tape stripping was performed five times daily for three consecutive days from Day 0 to Day 2. According to the randomization table, three of the areas were designated as sample sites and treated with the test product once daily, while the remaining one area served as the model control and did not receive the test product.

### 3.4 Test Environment

The skin on the flexor side of the arm was exposed, and the subjects were acclimated for 20 minutes in an environment with a temperature of 20-22°C and a relative humidity of 40-60% before conducting the skin measurements.

### 3.5 Test Procedure

On Day 0, after signing the informed consent form, subjects underwent screening, and those who met the screening criteria were enrolled in the study. Four 3cm*3cm areas were marked on the flexor side of the arm as test sites. After acclimatization in a temperature- and humidity-controlled environment for 20 minutes, the stratum corneum moisture content and TEWL of the test sites were measured. Subsequently, tape stripping was performed on the test sites, with five strips taken from each site. On Days 1-2, tape stripping was similarly conducted on the test sites, with five strips taken from each site daily. After tape stripping, the test product was applied to the sample sites at a dosage of approximately 2 mg/$cm^2$, while the model control site remained untreated. From Day 3 to Day 7, the test product was applied to the sample sites at a dosage of approximately 2 mg/$cm^2$ daily, while the model control site remained untreated. Measurements of the stratum corneum moisture content and TEWL of the test sites were taken after acclimatization in the temperature- and humidity-controlled environment for 20 minutes on Day 2, post-tape stripping, and Day 7, respectively.

### 3.6 Adverse Reactions

If any adverse reactions occur in the volunteers, the use of the test product should be immediately discontinued, and the individual should visit the research center for examination by a physician. The physician will determine whether to terminate the use of the sample based on the circumstances. Any adverse reactions and other related events that occur during the test period should be documented and reflected in the report.

### 3.7 Data Analysis

The average value and standard deviation of each measurement indicator at each time point for all subjects were calculated. The barrier repair rate of each test group was calculated based on the change rate of the TEWL value of each test group after the end of treatment (Day 7) relative to the TEWL value on Day 0 (baseline TEWL value) and the TEWL value after tape stripping on Day 2. The calculation formula is as follows:

$$\text{Barrier repair rate (\%)} = (T_2 - T_7) / (T_2 - T_0) \times 100\%,$$

where $T_2$ is the TEWL value measured immediately after tape stripping on Day 2, $T_7$ is the TEWL value of each test group

after treatment on Day 7, and $T_0$ is the baseline TEWL value measured before tape application on Day 0.

Statistical analysis of the data was performed using SPSS 22.0 software. A one-way ANOVA was conducted, followed by Dunnett's post-hoc test for pairwise comparisons.

## 4 Test Results

A total of 32 eligible subjects were recruited for this trial, including 3 males and 29 females, with ages ranging from 24 to 58 years old, and an average age of 41.2±10.7 years. No adverse reactions were reported during the trial. The test results for TEWL and stratum corneum moisture content in the test areas of each test group are presented below.

### 4.1 Transepidermal Water Loss (TEWL) Values of Each Test Group

The changing trend of TEWL values in the test area of each test group is shown in Figure 10:
($^{\#}p<0.05$ compared with the normal skin control group, $^{*}p<0.05$ compared with the blank hydrogel matrix group)

The test results of TEWL values for the test areas of each test group indicate that the three consecutive days of tape stripping led to an increase in TEWL values in each test area, from a baseline value of ~5 g/($m^2$•h) to ~13 g/($m^2$•h) after tape stripping on Day 2, demonstrating significant impairment of the epidermal barrier function caused by tape stripping. After tape stripping, the test products were applied once daily to the test areas until Day 7. The TEWL values in the test areas of each test group were significantly reduced compared to those after tape stripping on Day 2, indicating a certain degree of repair of the impaired epidermal barrier. Moreover, the areas treated with the test samples "0.1% α-ionone hydrogel" and "1% α-ionone hydrogel" exhibited a greater reduction in TEWL values, and their differences in TEWL values compared with the test group using the "blank hydrogel matrix" and the model group that underwent tape stripping but did not use the test samples. This suggests that the use of the test samples "0.1% α-ionone hydrogel" and "1% α-ionone hydrogel" can accelerate the repair of the epidermal barrier damaged by tape stripping.

### 4.2 Stratum Corneum Moisture Content of Each Test Group

The changing trend of the stratum corneum moisture content in the test area of each test group is shown in Figure 11:
($^{*}p<0.05$ compared with the blank hydrogel matrix group, $^{\#}p<0.05$ compared with the normal skin control group)

The test results of the stratum corneum moisture content in the test areas of each test group indicate that three consecutive days of tape stripping did not cause significant changes in the stratum corneum moisture content of the test areas. However, on Day 7, the model group (which underwent tape stripping but did not use the test samples) showed a significant decrease in stratum corneum moisture content compared to the baseline level, with an average reduction of 9.13%. This suggests that the increased transepidermal water loss due to tape stripping led to skin dryness. In contrast, the test areas treated with the test samples "blank hydrogel matrix", "0.1% α-ionone hydrogel" and "1% α-ionone hydrogel" also experienced a decrease in stratum corneum moisture content compared to the baseline, but the reduction was less pronounced, with average decreases of 7.71%, 3.99%, and 2.81%, respectively. The stratum corneum moisture content in the area treated with the test sample "blank hydrogel matrix" was not significantly different from that of the model group ($p\geq0.05$). However, the areas treated with the test samples "0.1% α-ionone hydrogel" and "1% α-ionone hydrogel" showed significantly higher stratum corneum moisture content compared to the model group ($p<0.05$). This further suggests that the use of the test samples "0.1% α-ionone hydrogel" and "1% α-ionone hydrogel" can accelerate the repair of the epidermal barrier damaged by tape stripping, thereby increasing the moisture content of the stratum corneum in the test areas.

### 4.3 Barrier Repair Rate of Each Test Group

The barrier repair rates calculated based on the change rate of TEWL values for each test group are shown in Figure 12:
($^{*}p<0.05$ compared with the blank hydrogel matrix group, $^{**}p<0.01$ compared with the blank hydrogel matrix group)

The results of the barrier repair rate calculated based on the change rate of the TEWL value of each test group further indicate that the barrier repair rate of the test group using the test sample "blank hydrogel matrix" did not show a significant difference compared with the model group that did not use the test samples. In contrast, the test groups using the test samples "0.1% α-ionone hydrogel" and "1% α-ionone hydrogel" exhibited a significant increase in barrier repair rate compared with both the model group and the "blank hydrogel matrix" group. This result demonstrates that the use of the test samples "0.1% α-ionone hydrogel" and "1% α-ionone hydrogel" can indeed accelerate the repair of the epidermal barrier damaged by tape stripping, thus confirming their reparative efficacy.

**5 Conclusion**

The test results of this trial indicate that the test sample "blank hydrogel matrix" does not possess reparative efficacy, whereas the "0.1% α-ionone hydrogel" and "1% α-ionone hydrogel" do exhibit reparative efficacy.

**Efficacy Testing of Cosmetics**

To verify the efficacy of the cosmetics of the present invention, the Inventors conducted efficacy tests on the toner, emulsion, and cream of Examples 1 to 6, as detailed below.

**A Test Samples:**

| No. | Sample Name | Notes |
|---|---|---|
| 1 | Water 1 | Blank control water (without α-ionone) |
| 2 | Water 2 | Formula of Example 1 |
| 3 | Water 3 | Formula of Example 2 |
| 4 | Emulsion 1 | Blank control emulsion (without α-ionone) |
| 5 | Emulsion 2 | Formula of Example 3 |
| 6 | Emulsion 3 | Formula of Example 4 |
| 7 | Cream 1 | Blank control cream (without α-ionone) |
| 8 | Cream 2 | Formula of Example 5 |
| 9 | Cream 3 | Formula of Example 6 |

**B. Test Methods**

**B.1 Subjects**

**B.1.1 Target Number of Subjects: 30 individuals.**

**B.1.2 Inclusion Criteria**

Aged between 30 and 50 years old;
Even skin tone on the arms, with no skin issues such as folliculitis or eczema that may affect the test;
Consent to undergo tape stripping on the arm skin;
No use of any other products on the test area during the test period;
Informed consent, with the ability to use the products and complete the required tests as instructed by the researchers.

**B.1.3 Exclusion Criteria□**

Pregnant or breastfeeding women;
Individuals with severe systemic diseases or those currently using systemic medications for such conditions;
Individuals who have undergone skin treatments, cosmetic procedures, or other tests on the test site that may affect the results;
Individuals with allergic diseases or a history of allergies to cosmetics;
Individuals who are currently participating in or have participated in other clinical trials prior to the start of this study.

### B.1.4 Dropout criteria

Subjects lost to follow-up or actively requested to withdraw;
Poor compliance, such as failure to use the test products as instructed, or failure to attend follow-up visits as required;
Use of other cosmetics similar to the test products during the study;
Development of any new disease that directly affects the clinical status assessment, including skin diseases, during the study period;
Development of a serious illness during the study period;
Pregnancy during the study period;
Inability to tolerate the study procedures;
Occurrence of a serious adverse event (SAE) during the study period.

### B.2 Instruments

A device for measuring transepidermal water loss (Tewameter, C&K, Germany);
A device for measuring stratum corneum moisture content (Corneometer, C&K, Germany).

### B.3 Test indicators

**B.3.1 Stratum Corneum Moisture Content** Stratum corneum moisture content is one of the evaluation indicators for the reparative efficacy of cosmetics. A higher value of this indicator indicates a higher moisture content in the stratum corneum. After the skin barrier is slightly impaired by tape stripping, if the stratum corneum moisture content in the area treated with the product is significantly higher compared to the untreated area or control area, it can be concluded that the product has reparative efficacy.

B.3.2 The rate of transepidermal water loss (TEWL) is one of the indicators for evaluating the repair efficacy of cosmetics. The higher the value of this indicator, the faster the rate of water loss from the skin surface, and the poorer the skin barrier function. After the skin barrier is mildly damaged by tape stripping, if the TEWL in the area where the product is used is significantly lower than that in the area where the product is not used or in the control area, it can be concluded that the product has a repair efficacy.

### B.4 Measurement Sites

The measurement sites for the moisture content of the stratum corneum and TEWL are both in the area on the inner side of the arm where the skin barrier is mildly damaged by tape stripping.

### B.5 Test Environment

The skin of the measurement sites was exposed, and the subjects were acclimated for 20 minutes in an environment with a temperature of 20-22°C and a relative humidity of 40-60% before conducting the skin measurements.

### B.6 Test Procedure

(1) On Day 0, after signing the informed consent form, subjects underwent screening, and those who met the screening criteria were enrolled in the study. After being cleaned with soapy water, the flexor side of the arm was marked with eleven 3cm*3cm areas as test sites. After acclimatization in a temperature- and humidity-controlled environment for 20 minutes, the stratum corneum moisture content and TEWL of the test sites were measured. Subsequently, tape stripping was performed on ten of the test sites, with ten strips taken from each site, while the remaining area was left untreated as a blank control. After 15 minutes, the moisture content of the stratum corneum and TEWL of the test areas were measured again. Based on the randomization table, the product was applied quantitatively to 9 of the tape-stripped areas at a dosage of approximately 2mg/$cm^2$, while one tape-stripped area was left untreated as a model control. Six hours later, the moisture content of the stratum corneum and TEWL of the test areas were measured again.

(2) On Days 1 to 9, the product was applied twice daily to the test areas, with each application being approximately 2mg/cm$^2$. The model control and blank control areas did not receive any product application.
(3) On Day 1, Day 3, Day 7, and Day 10, the skin on the flexor side of the arm was cleaned with soapy water. After acclimatization in a temperature- and humidity-controlled environment for 20 minutes, the stratum corneum moisture content and TEWL of the test sites were measured.

**B.7 Adverse Reactions**

If any adverse reactions occur in the volunteers, the use of the test product should be immediately discontinued, and the individual should visit the research center for examination by a physician. The physician will determine whether to terminate the use of the sample based on the circumstances. Any adverse reactions and other related events that occur during the test period should be documented and reflected in the report.

**B.8 Data Analysis**

The average value and standard deviation of each measurement indicator at each time point for all subjects were calculated. For the stratum corneum moisture content, the change value and change rate relative to pre-stripping measurements were calculated using the following formulas:

$$\text{Change value }(\Delta) = T_n - T_0$$

$$\text{Change rate} = \frac{T_n - T_0}{T_0} * 100\%$$

In the formula,

$T_0$-The measured value of the indicator before stripping
$T_n$-The measured value of the indicator at the n$^{th}$ follow-up visit
n-The number of follow-up visits

For TEWL, the repair rate for each location was calculated using the following formula:

$$\text{Repair rate} = \frac{T_c - T_s}{T_c - T_b} * 100\%$$

In the formula,

$T_0$-The TEWL value immediately after tape stripping
$T_s$-The TEWL value after the application of the product
$T_b$-The TEWL value before tape stripping

The data were statistically analyzed using SPSS 22.0 software. One-way analysis of variance was employed for inter-group testing, and the LSD method was used for pairwise comparisons. All tests were two-tailed, with a significance level of $\alpha$=0.05.

**C Test Results**

This trial recruited 30 subjects, all with Chinese skin types, including 5 males and 25 females, aged between 30 and 50 years, with an average age of 38.0±5.5 years. All 30 subjects completed the entire test. The test results are as follows.

**C.1 Stratum Corneum Moisture Content**

Stratum corneum moisture content is one of the evaluation indicators for the reparative efficacy of cosmetics. A higher value of this indicator indicates a higher moisture content in the stratum corneum. After the skin barrier is slightly impaired by tape stripping, if the stratum corneum moisture content in the area treated with the product is significantly higher compared to the untreated area or control area, it can be concluded that the product has reparative efficacy.

The measured moisture content values of the stratum corneum of the subjects are shown in Figure 13; in Figure 13, a: three water samples, b: three emulsion samples, c: three cream samples; the measured values of the model control and the blank control were analyzed for significance, with #$p<0.05$; the measured values of Sample 2 and Sample 3 were compared with those of Sample 1 for significance analysis, with *$p<0.05$.

The changes in the stratum corneum moisture content of the subjects compared to pre-stripping values are shown in Figure 14. In Figure 14, a: three aqueous samples, b: three emulsion samples, c: three cream samples. The changes of the model control and the blank control were analyzed for significance, with # $p<0.05$; the changes of Sample 2 and Sample 3 were compared with those of Sample 1 for significance analysis, with *$p<0.05$.

The results of the change rate in the average stratum corneum moisture content relative to pre-stripping values during the testing period are presented in Table 1.

Table 1 Results of the Relative Change Rate in Average Stratum Corneum Moisture Content Relative to Pre-stripping Values

| Group | Before Stripping | Immediately After Stripping | 6 Hours | 1 Day | 3 Days | 7 Days | 10 Days |
|---|---|---|---|---|---|---|---|
| Blank Control | / | -3.4% | -4.6% | -4.6% | -4.7% | -2.0% | 0.6% |
| Model Control | / | 128.6% | 71.0% | 29.2% | -36.2% | -24.7% | -13.5% |
| Water 1 | / | 116.9% | 84.0% | 25.0% | -27.7% | -18.0% | -1.6% |
| Water 2 | / | 127.5% | 94.3% | 33.3% | -27.0% | -12.0% | 7.2% |
| Water 3 | / | 123.3% | 92.4% | 34.7% | -23.1% | -2.1% | 16.5% |
| Emulsion 1 | / | 130.4% | 91.8% | 35.3% | -23.7% | -6.5% | 15.3% |
| Emulsion 2 | / | 126.4% | 98.6% | 38.1% | -24.9% | -2.8% | 18.6% |
| Emulsion 3 | / | 127.5% | 111.1% | 39.7% | -22.6% | 3.9% | 25.9% |
| Cream 1 | / | 127.9% | 92.3% | 36.2% | -22.1% | -1.1% | 21.6% |
| Cream 2 | / | 129.1% | 95.8% | 38.7% | -21.6% | 3.1% | 25.2% |
| Cream 3 | / | 124.1% | 95.5% | 43.5% | -14.4% | 12.8% | 35.7% |

Result Description:

(1) Before tape stripping, there were no significant differences in the moisture content of the stratum corneum across all tested areas.

(2) Immediately after tape stripping, the relative change rates of the moisture content in the stratum corneum for the blank control, model control, Water 1, Water 2, Water 3, Emulsion 1, Emulsion 2, Emulsion 3, Cream 1, Cream 2, and Cream 3 areas were -3.4%, 128.6%, 116.9%, 127.5%, 123.3%, 130.4%, 126.4%, 127.5%, 127.9%, 129.1%, and 124.1%, respectively. The moisture content in the stratum corneum of the areas subjected to tape stripping was significantly higher compared to the blank control. There were no significant differences among the areas that underwent tape stripping.

(3) Six hours after the first application of the product following tape stripping, the relative change rates of the moisture content in the stratum corneum for the blank control, model control, Water 1, Water 2, and Water 3 areas were -4.6%, 71.0%, 84.0%, 94.3%, and 92.4%, respectively.

On Day 1 of the trial, the relative change rates of the moisture content in the stratum corneum for the blank control, model control, Water 1, Water 2, and Water 3 areas were -4.6%, 29.2%, 25.0%, 33.3%, and 34.7%, respectively.

On Day 3 of the trial, the relative change rates of the moisture content in the stratum corneum for the blank control, model control, Water 1, Water 2, and Water 3 areas were -4.7%, -36.2%, -27.7%, -27.0%, and -23.1%, respectively.

On Day 7 of the trial, the relative change rates of the moisture content in the stratum corneum for the blank control, model control, Water 1, Water 2, and Water 3 areas were -2.0%, -24.7%, -18.0%, -12.0%, and -2.1%, respectively; the moisture content in the stratum corneum of Water 2 was higher than that of Water 1, and the moisture content in the stratum corneum of Water 3 was significantly higher than that of Water 1; the change in the moisture content in the stratum corneum for Water 2 was greater than that for Water 1, and the change in the moisture content in the stratum corneum for Water 3 was significantly greater than that for Water 1.

On Day 10 of the trial, the relative change rates of the moisture content in the stratum corneum for the blank control, model control, Water 1, Water 2, and Water 3 areas were -0.6%, -13.5%, -1.6%, 7.2%, and 16.5%, respectively; the

moisture content in the stratum corneum of Water 2 was higher than that of Water 1, and the moisture content in the stratum corneum of Water 3 was significantly higher than that of Water 1; the change in the moisture content in the stratum corneum for Water 2 was greater than that for Water 1, and the change in the moisture content in the stratum corneum for Water 3 was significantly greater than that for Water 1.

(4) Six hours after the first use of the product following tape stripping, the relative change rates of the moisture content in the stratum corneum for the blank control, model control, Emulsion 1, Emulsion 2, and Emulsion 3 areas were -4.6%, 71.0%, 91.8%, 98.6%, and 111.1%, respectively.

On Day 1 of the trial, the relative change rates of the moisture content in the stratum corneum for the blank control, model control, Emulsion 1, Emulsion 2, and Emulsion 3 areas were -4.6%, 29.2%, 35.3%, 38.1%, and 39.7%, respectively.

On Day 3 of the trial, the relative change rates of the moisture content in the stratum corneum for the blank control, model control, Emulsion 1, Emulsion 2, and Emulsion 3 areas were -4.7%, -36.2%, -23.7%, -24.9%, and -22.6%, respectively.

On Day 7 of the trial, the relative change rates of the moisture content in the stratum corneum for the blank control, model control, Emulsion 1, Emulsion 2, and Emulsion 3 areas were -2.0%, -24.7%, -6.5%, -2.8%, and 3.9%, respectively; the moisture content in the stratum corneum of Emulsion 2 was higher than that of Emulsion 1, and the moisture content in the stratum corneum of Emulsion 3 was significantly higher than that of Emulsion 1.

On Day 10 of the trial, the relative change rates of the moisture content in the stratum corneum for the blank control, model control, Emulsion 1, Emulsion 2, and Emulsion 3 areas were -0.6%, -13.5%, 15.3%, 18.6%, and 25.9%, respectively; the moisture content in the stratum corneum of Emulsion 2 was higher than that of Emulsion 1, and the moisture content in the stratum corneum of Emulsion 3 was significantly higher than that of Emulsion 1.

(5) Six hours after the first application of the product following tape stripping, the relative change rates of the moisture content in the stratum corneum for the blank control, model control, Cream 1, Cream 2, and Cream 3 areas were -4.6%, 71.0%, 92.3%, 95.8%, and 95.5%, respectively.

On Day 1 of the trial, the relative change rates of the moisture content in the stratum corneum for the blank control, model control, Cream 1, Cream 2, and Cream 3 areas were - 4.6%, 29.2%, 36.2%, 38.7%, and 43.5%, respectively.

On Day 3 of the trial, the relative change rates of the moisture content in the stratum corneum for the blank control, model control, Cream 1, Cream 2, and Cream 3 areas were - 4.7%, -36.2%, -22.1%, -21.6%, and -14.4%, respectively.

On Day 7 of the trial, the relative change rates of the moisture content in the stratum corneum for the blank control, model control, Cream 1, Cream 2, and Cream 3 areas were - 2.0%, -24.7%, -1.1%, 3.1%, and 12.8%, respectively; the moisture content in the stratum corneum of Cream 2 was higher than that of Cream 1, and the moisture content in the stratum corneum of Cream 3 was significantly higher than that of Cream 1; the change in the moisture content in the stratum corneum for Cream 2 was greater than that for Cream 1, and the change in the moisture content in the stratum corneum for Cream 3 was significantly greater than that for Cream 1.

On Day 10 of the trial, the relative change rates of the moisture content in the stratum corneum for the blank control, model control, Cream 1, Cream 2, and Cream 3 areas were - 0.6%, -13.5%, 21.6%, 25.2%, and 35.7%, respectively; the moisture content in the stratum corneum of Cream 2 was higher than that of Cream 1, and the moisture content in the stratum corneum of Cream 3 was significantly higher than that of Cream 1; the change in the moisture content in the stratum corneum for Cream 2 was greater than that for Cream 1, and the change in the moisture content in the stratum corneum for Cream 3 was significantly greater than that for Cream 1.

C.2 Transepidermal Water Loss Rate (TEWL)

The rate of transepidermal water loss (TEWL) is one of the indicators for evaluating the repair efficacy of cosmetics. The higher the value of this indicator, the faster the rate of water loss from the skin surface, and the poorer the skin barrier function. After the skin barrier is mildly damaged by tape stripping, if the TEWL in the area where the product is used is significantly lower than that in the area where the product is not used or in the control area, it can be concluded that the product has a repair efficacy.

The measured TEWL values for the test areas of the subjects are shown in Figure 15; in Figure 15, a: three water samples, b: three emulsion samples, c: three cream samples; the measured values of the model control and the blank control were analyzed for significance, with # $p<0.05$; the measured values of Sample 2, Sample 3 and Sample 1 were analyzed for significance, with *$p<0.05$.

The TEWL repair rate of the subjects is shown in Figure 16; in Figure 16, a: three water samples, b: three emulsion samples, c: three cream samples; the repair rates of Sample 2, Sample 3 and Sample 1 were analyzed for significance, with *$p<0.05$.

Result Description:

(1) Before tape stripping, there were no significant differences in the TEWL across all tested areas.

(2) Immediately after tape stripping, the TEWL in the areas subjected to tape stripping was significantly higher compared to the blank control, and there were no significant differences among the areas that underwent tape stripping.

(3) Six hours after the first application of the product following tape stripping, the TEWL repair rates for the model control, Water 1, Water 2, and Water 3 were 1.7%, 8.0%, 4.1%, and 2.2%, respectively.

On Day 1 of the trial, the TEWL repair rates for the model control, Water 1, Water 2, and Water 3 were 8.9%, 22.5%, 21.3%, and 19.5%, respectively.

On Day 3 of the trial, the TEWL repair rates for the model control, Water 1, Water 2, and Water 3 were 41.3%, 49.0%, 47.3%, and 51.4%, respectively.

On Day 7 of the trial, the TEWL repair rates for the model control, Water 1, Water 2, and Water 3 were 74.4%, 83.2%, 82.5%, and 82.6%, respectively.

On Day 10 of the trial, the TEWL repair rates for the model control, Water 1, Water 2, and Water 3 were 79.4%, 84.8%, 87.2%, and 87.2%, respectively.

(4) Six hours after the first use of the product following tape stripping, the TEWL repair rates for the model control, Emulsion 1, Emulsion 2, and Emulsion 3 were 1.7%, 7.4%, 4.5%, and 4.2%, respectively.

On Day 1 of the trial, the TEWL repair rates for the model control, Emulsion 1, Emulsion 2, and Emulsion 3 were 8.9%, 20.0%, 11.4%, and 15.0%, respectively.

On Day 3 of the trial, the TEWL repair rates for the model control, Emulsion 1, Emulsion 2, and Emulsion 3 were 41.3%, 48.5%, 39.3%, and 51.2%, respectively.

On Day 7 of the trial, the TEWL repair rates for the model control, Emulsion 1, Emulsion 2, and Emulsion 3 were 74.4%, 75.6%, 76.2%, and 83.3%, respectively.

On Day 10 of the trial, the TEWL repair rates for the model control, Emulsion 1, Emulsion 2, and Emulsion 3 were 79.4%, 83.8%, 80.1%, and 84.0%, respectively.

(5) Six hours after the first application of the product following tape stripping, the TEWL repair rates for the model control, Cream 1, Cream 2, and Cream 3 were 1.7%, 6.9%, -7.6%, and -0.6%, respectively. On Day 1 of the trial, the TEWL repair rates for the model control, Cream 1, Cream 2, and Cream 3 were 8.9%, 14.9%, 6.6%, and 13.5%, respectively.

On Day 3 of the trial, the TEWL repair rates for the model control, Cream 1, Cream 2, and Cream 3 were 41.3%, 43.1%, 42.3%, and 44.3%, respectively.

On Day 7 of the trial, the TEWL repair rates for the model control, Cream 1, Cream 2, and Cream 3 were 74.4%, 75.8%, 80.0%, and 82.7%, respectively.

On Day 10 of the trial, the TEWL repair rates for the model control, Cream 1, Cream 2, and Cream 3 were 79.4%, 80.5%, 81.5%, and 85.9%, respectively.

## D Conclusion

In summary, the repair efficacy of the test sample Water 2 is better than that of Water 1, and Water 3 has a more significant repair effect than that of Water 1; the repair efficacy of Emulsion 2 is better than that of Emulsion 1, and Emulsion 3 has a more significant repair effect than that of Emulsion 1; the repair efficacy of Cream 2 is better than that of Cream 1, and Cream 3 has a more significant repair effect than that of Cream 1.

## Industrial Applicability

The inventors of the present patent have discovered that α-ionone has a regulatory effect on the functions of keratinocytes, promoting the proliferation and migration of HaCaT cells, and mitigating the inhibitory effect of norepinephrine on cell migration. α-Ionone can also induce the expression of HAS-2 and HBD-2 genes, facilitating the synthesis of HA and HBD-2 by HaCaT cells, and reducing the inhibitory effect of dexamethasone on the expression of HAS-2 and HBD-2. The aforementioned findings of the inventors suggest that α-ionone may possess the efficacy of promoting skin injury repair.

Therefore, the present invention enables the use of α-ionone as an active ingredient in skin barrier repair agents for topical skin applications, which can also serve as a repair agent for skin barrier dysfunction caused by the use of glucocorticoids and/or psychological stress. When incorporated into topical skin formulations, particularly cosmetics, α-ionone serves to repair the skin barrier, for instance, by moisturizing the skin, promoting the repair of damaged skin, and facilitating the repair of skin barrier impairment caused by topical glucocorticoids and stress.

## Claims

1. A skin barrier function repair agent for use in topical skin formulations, **characterized in that** it comprises α-ionone as an active ingredient acting on keratinocytes.

2. The skin barrier function repair agent according to claim 1, **characterized in that** the α-ionone functions as a keratinocyte migration promoter.

3. The skin barrier function repair agent according to claim 1, **characterized in that** the α-ionone functions as a keratinocyte proliferation promoter.

4. The skin barrier function repair agent according to claim 1, **characterized in that** the α-ionone functions as a promoter of hyaluronan synthase-2 expression in keratinocytes.

5. The skin barrier function repair agent according to claim 1, **characterized in that** the α-ionone functions as a promoter of human β-defensin-2 expression in keratinocytes.

6. The skin barrier function repair agent according to claim 1, **characterized in that** it is used as a repair agent for skin barrier dysfunction caused by the use of glucocorticoids and/or psychological stress.

7. The skin barrier function repair agent according to claim 1, **characterized in that** the topical skin formulation is a cosmetic product.

8. Use of α-ionone in the preparation of cosmetics for promoting skin barrier repair, wherein the promotion of skin barrier repair is achieved through any of the following promoting actions: promoting the proliferation of keratinocytes, promoting the migration of keratinocytes, promoting the expression of HAS-2 in keratinocytes, and promoting the expression of HBD-2 in keratinocytes.

9. Use of α-ionone in the preparation of cosmetics suitable for skin in a state with skin atrophy and/or skin barrier dysfunction caused by the use of glucocorticoids and/or psychological stress.

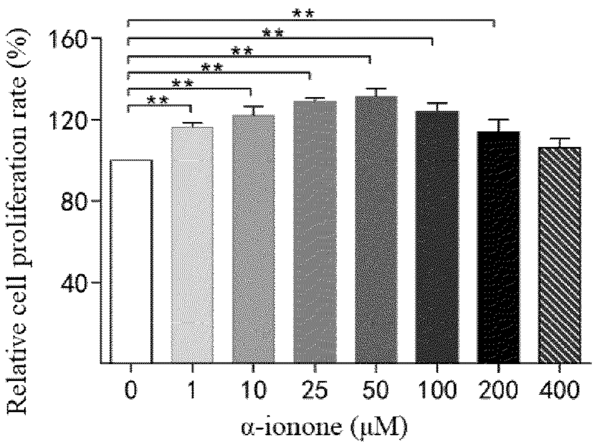


Figure 1

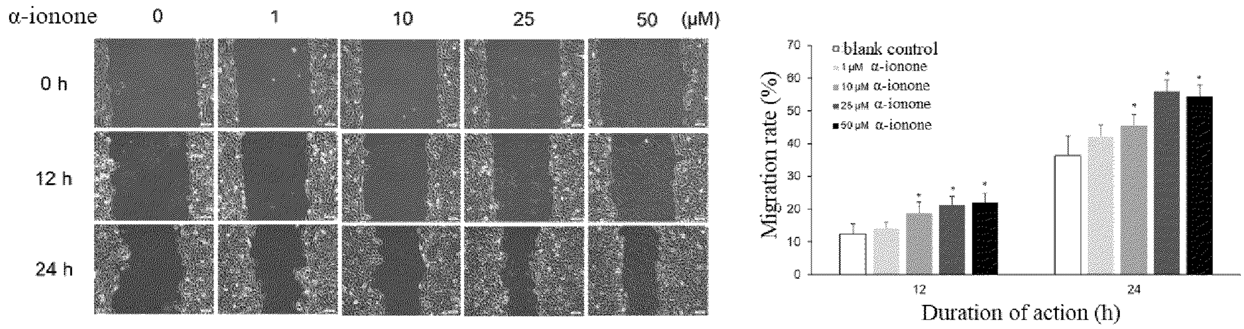


Figure 2

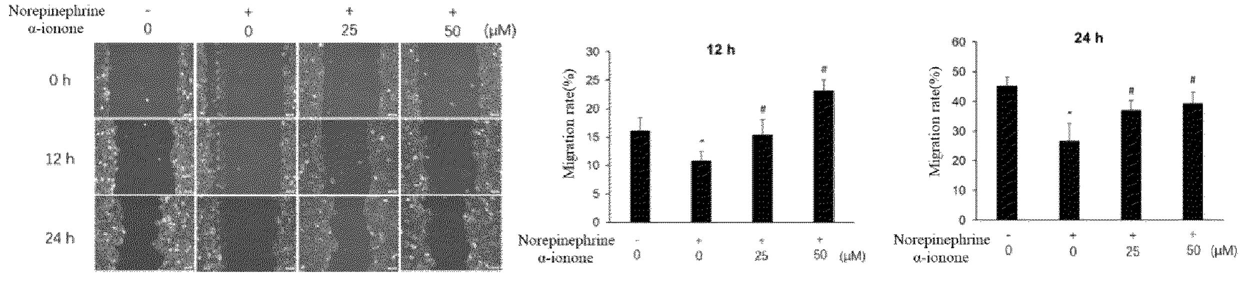


Figure 3

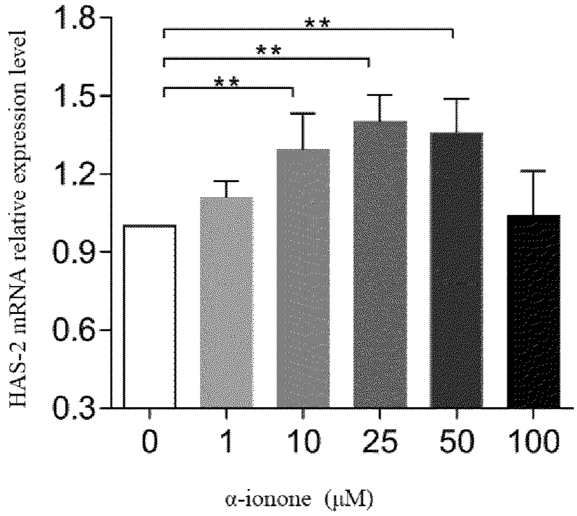


Figure 4

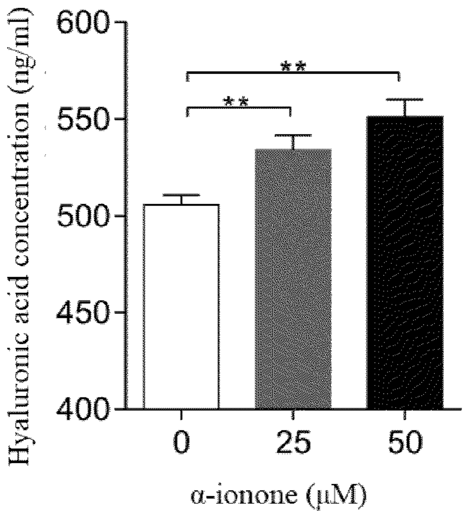


Figure 5

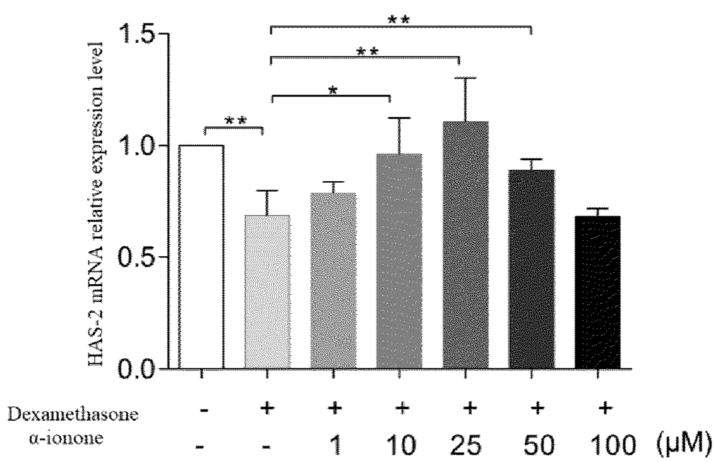


Figure 6

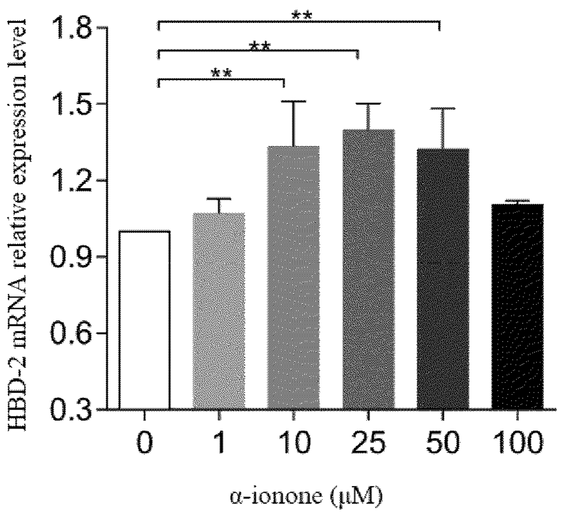


Figure 7

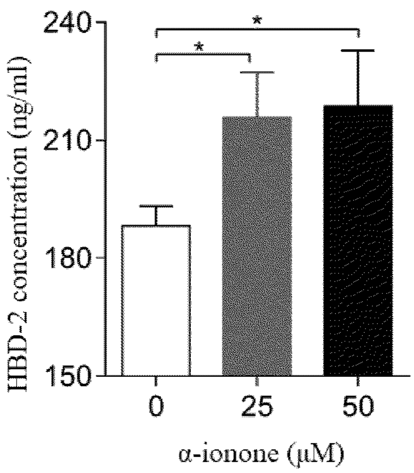


Figure 8

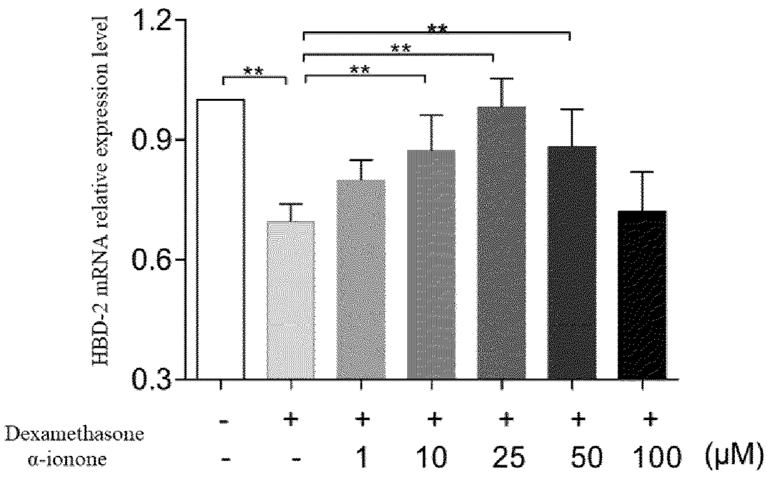


Figure 9

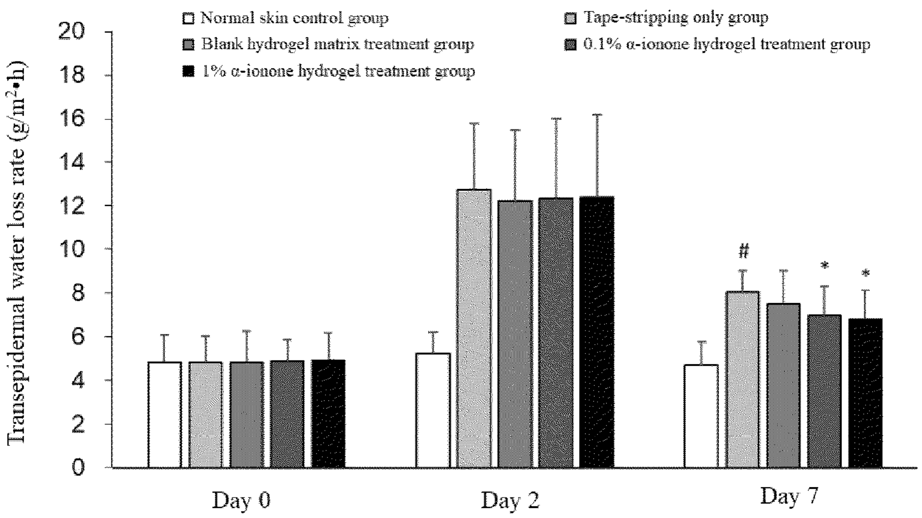


**Figure 10**

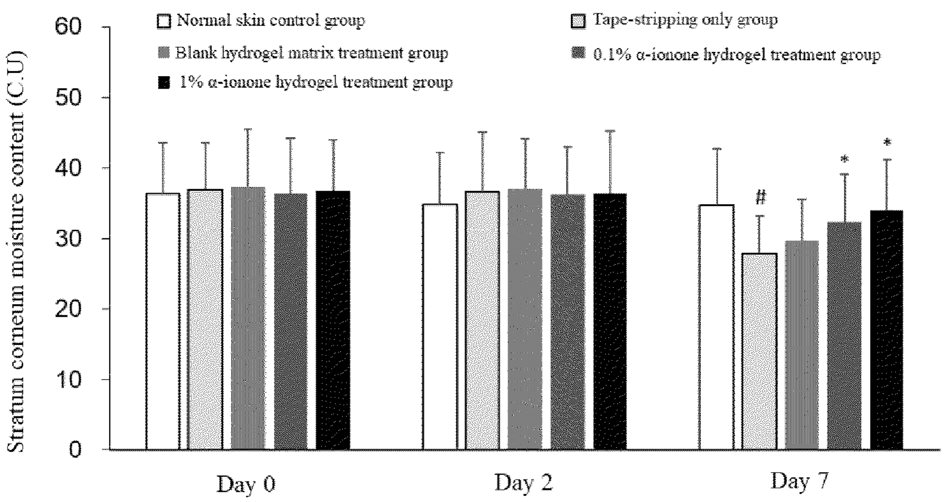


**Figure 11**

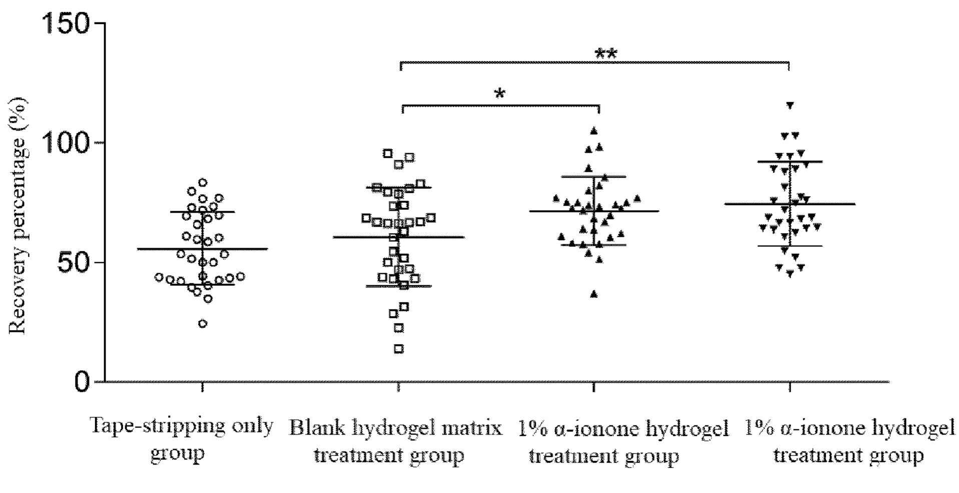


**Figure 12**

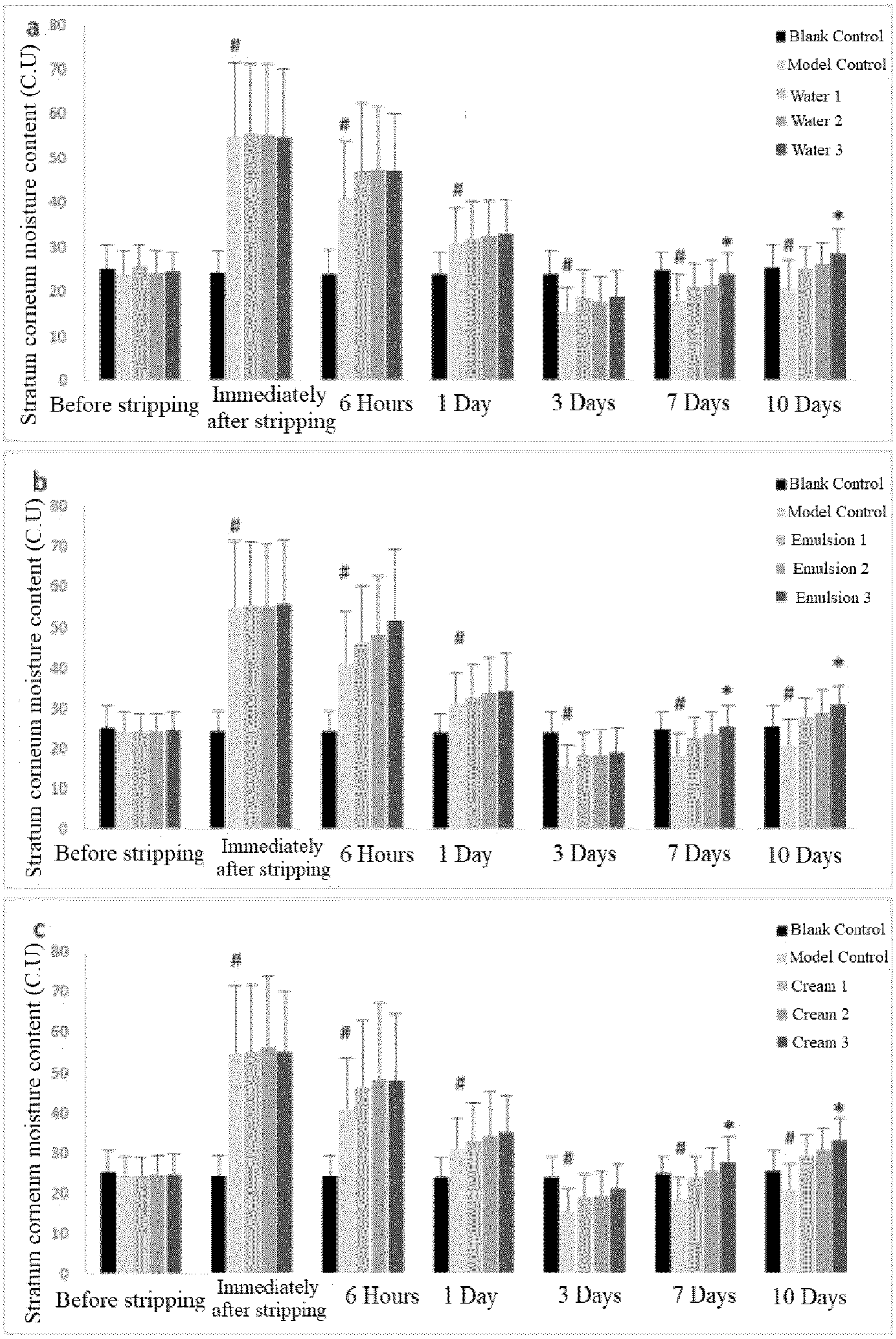
a
Stratum corneum moisture content (C.U)
0
10
20
30
40
50
60
70
80
Before stripping
Immediately after stripping
6 Hours
1 Day
3 Days
7 Days
10 Days
Blank Control
Model Control
Water 1
Water 2
Water 3
b
Stratum corneum moisture content (C.U)
Before stripping
Immediately after stripping
6 Hours
1 Day
3 Days
7 Days
10 Days
Blank Control
Model Control
Emulsion 1
Emulsion 2
Emulsion 3
c
Stratum corneum moisture content (C.U)
Before stripping
Immediately after stripping
6 Hours
1 Day
3 Days
7 Days
10 Days
Blank Control
Model Control
Cream 1
Cream 2
Cream 3

**Figure 13**

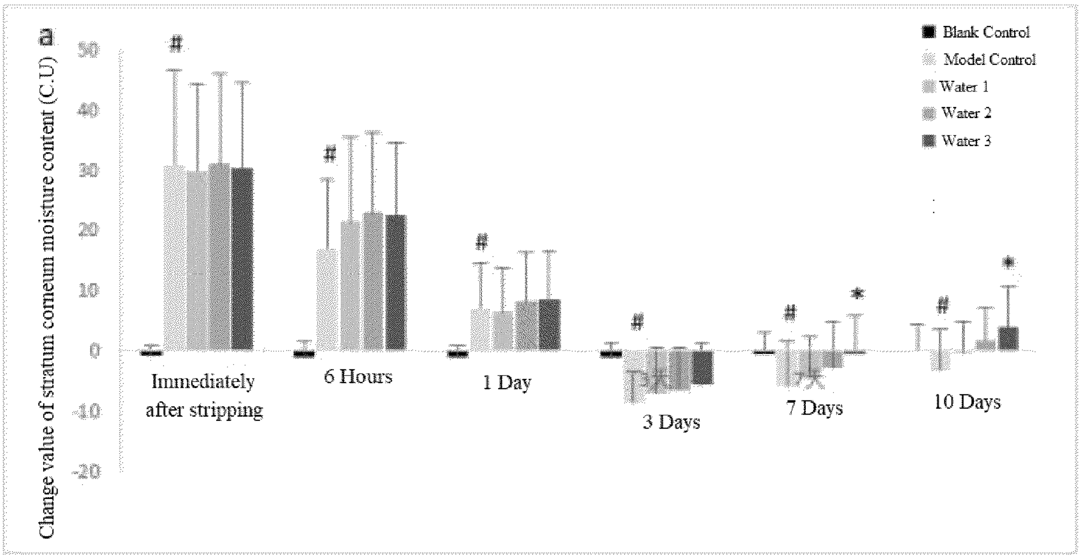
a
Change value of stratum corneum moisture content (C.U)
50
40
30
20
10
0
-10
-20
Blank Control
Model Control
Water 1
Water 2
Water 3
Immediately after stripping
6 Hours
1 Day
3 Days
7 Days
10 Days
#
*

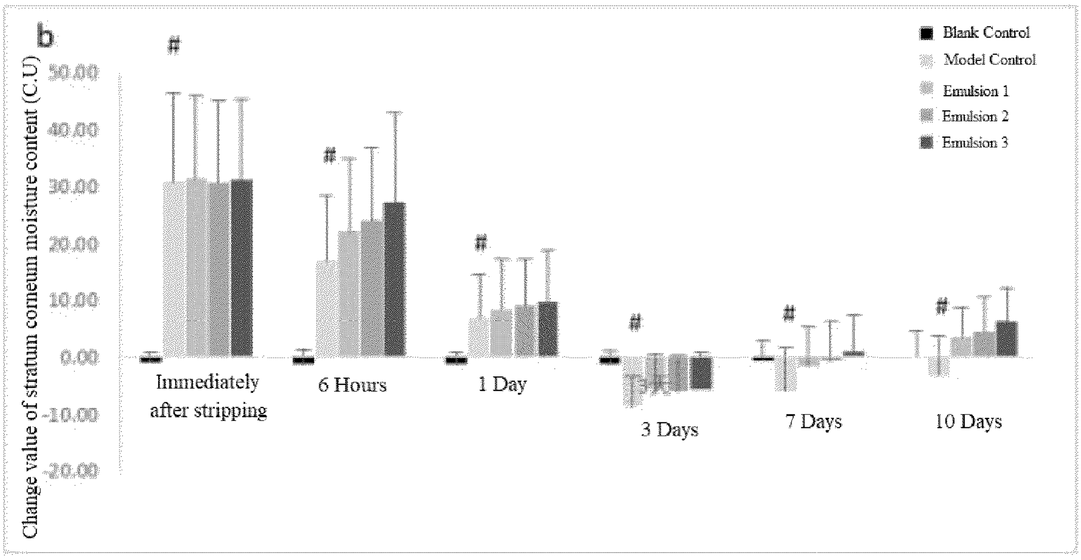
b
Change value of stratum corneum moisture content (C.U)
50.00
40.00
30.00
20.00
10.00
0.00
-10.00
-20.00
Blank Control
Model Control
Emulsion 1
Emulsion 2
Emulsion 3
Immediately after stripping
6 Hours
1 Day
3 Days
7 Days
10 Days
#

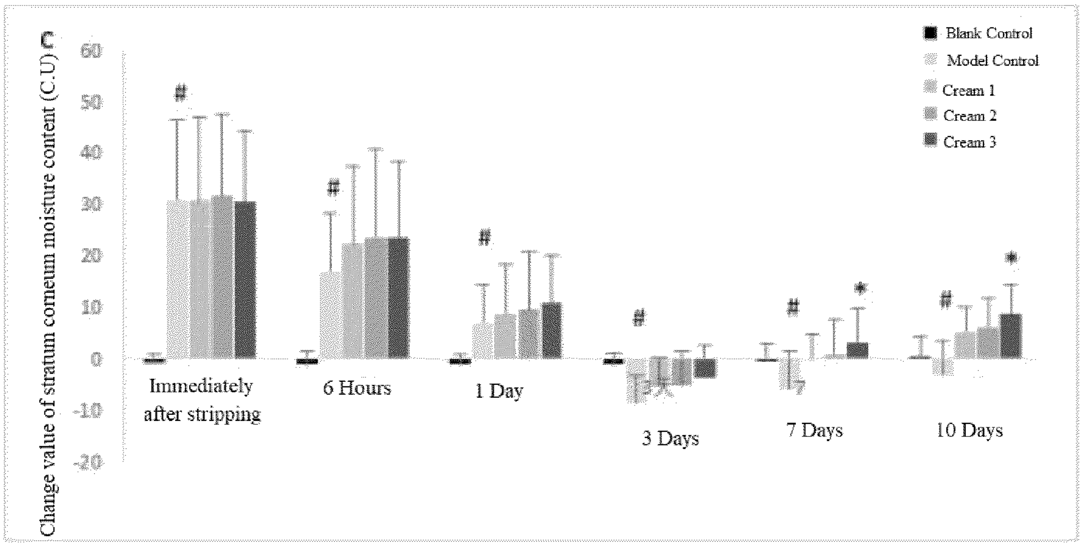
c
Change value of stratum corneum moisture content (C.U)
60
50
40
30
20
10
0
-10
-20
Blank Control
Model Control
Cream 1
Cream 2
Cream 3
Immediately after stripping
6 Hours
1 Day
3 Days
7 Days
10 Days
#
*

**Figure 14**

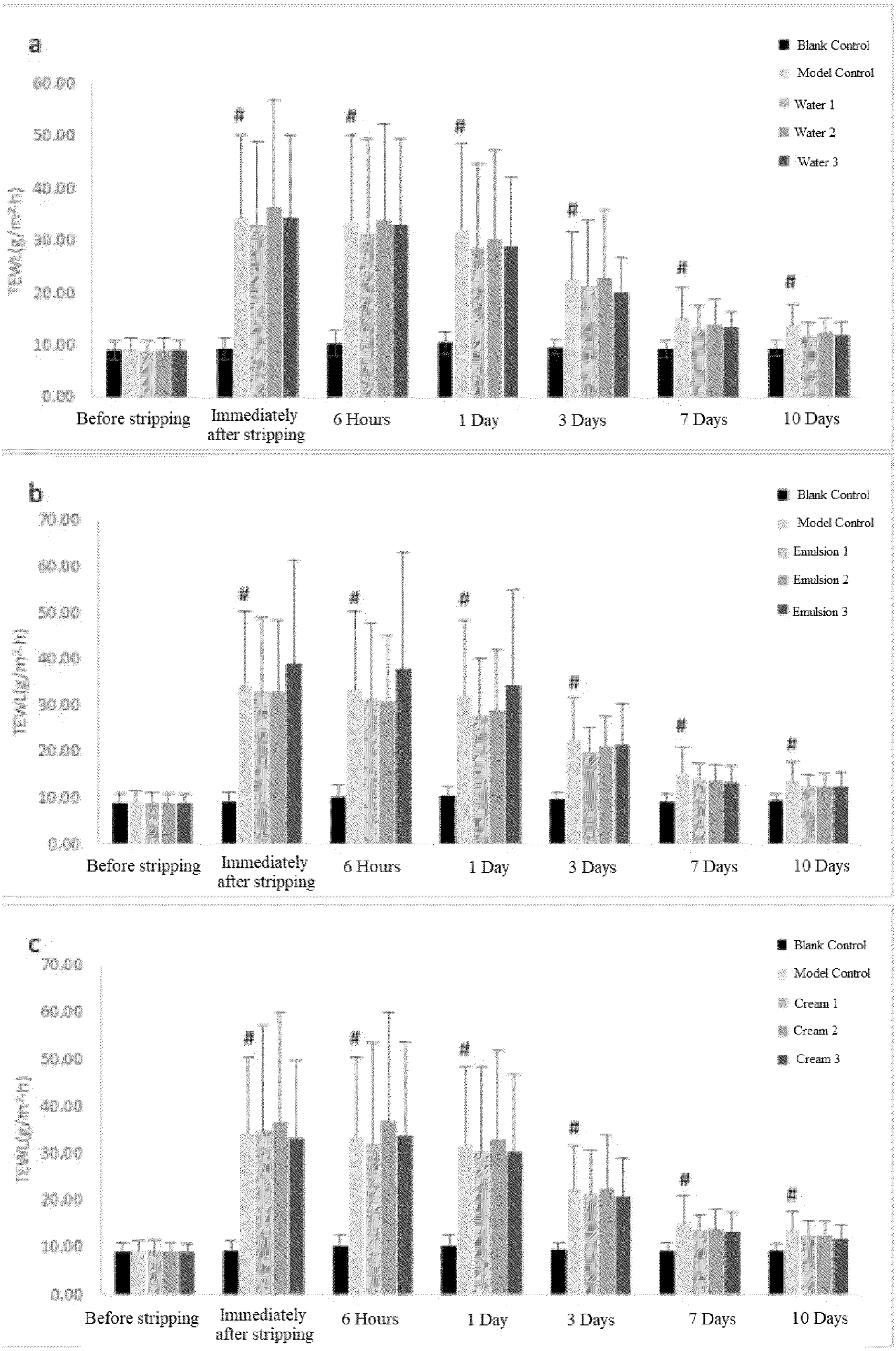
a
Blank Control
Model Control
Water 1
Water 2
Water 3
TEWL(g/m²·h)
60.00
50.00
40.00
30.00
20.00
10.00
0.00
Before stripping
Immediately after stripping
6 Hours
1 Day
3 Days
7 Days
10 Days
b
Blank Control
Model Control
Emulsion 1
Emulsion 2
Emulsion 3
TEWL(g/m²·h)
70.00
60.00
50.00
40.00
30.00
20.00
10.00
0.00
Before stripping
Immediately after stripping
6 Hours
1 Day
3 Days
7 Days
10 Days
c
Blank Control
Model Control
Cream 1
Cream 2
Cream 3
TEWL(g/m²·h)
70.00
60.00
50.00
40.00
30.00
20.00
10.00
0.00
Before stripping
Immediately after stripping
6 Hours
1 Day
3 Days
7 Days
10 Days

**Figure 15**

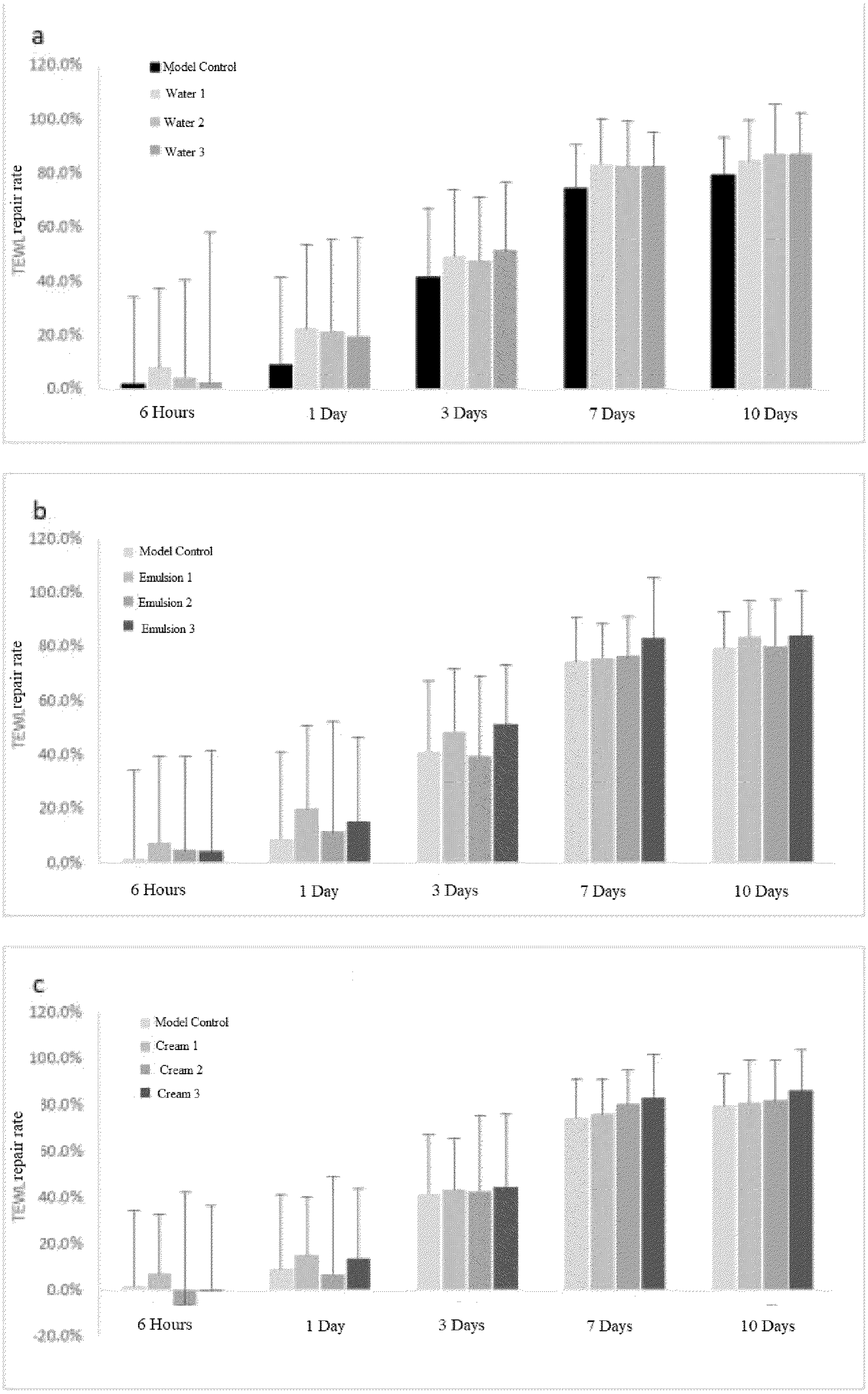
a
Model Control
Water 1
Water 2
Water 3
TEWL repair rate
120.0%
100.0%
80.0%
60.0%
40.0%
20.0%
0.0%
6 Hours
1 Day
3 Days
7 Days
10 Days
b
Model Control
Emulsion 1
Emulsion 2
Emulsion 3
TEWL repair rate
120.0%
100.0%
80.0%
60.0%
40.0%
20.0%
0.0%
6 Hours
1 Day
3 Days
7 Days
10 Days
c
Model Control
Cream 1
Cream 2
Cream 3
TEWL repair rate
120.0%
100.0%
30.0%
50.0%
40.0%
20.0%
0.0%
-20.0%
6 Hours
1 Day
3 Days
7 Days
10 Days

**Figure 16**

**INTERNATIONAL SEARCH REPORT**

International application No. **PCT/CN2023/137492**

**A. CLASSIFICATION OF SUBJECT MATTER**

A61K 8/35(2006.01)i; A61Q 19/00(2006.01)i; A61Q 19/08(2006.01)i

According to International Patent Classification (IPC) or to both national classification and IPC

**B. FIELDS SEARCHED**

Minimum documentation searched (classification system followed by classification symbols)

IPC: A61K A61Q

Documentation searched other than minimum documentation to the extent that such documents are included in the fields searched

Electronic data base consulted during the international search (name of data base and, where practicable, search terms used)

CNTXT; CNABS; ENTXTC; WPABSC; VEN; ENTXT; CJFD; CNKI; 万方, WANFANG; 读秀, DUXIU; ISI_Web of Science; Elsevier Science; SpringerLink; PubMed; STNext: 资生堂, 紫罗兰酮, 紫罗酮, 芷香酮, 环柠檬烯基丙酮, 基于紫罗兰酮的structural formula search, 皮肤, 肌肤, 表皮, 屏障, 角质形成细胞, 应激, 紧张, 压力, 糖皮质激素, Shiseido, ionone, skin, epidermal, barrier, keratinocyte, stress, pressure, glucocorticoid

**C. DOCUMENTS CONSIDERED TO BE RELEVANT**

| Category* | Citation of document, with indication, where appropriate, of the relevant passages | Relevant to claim No. |
|---|---|---|
| PX | YANG, Dandan et al. "α-Ionone Promotes Keratinocyte Functions and Accelerates Epidermal Barrier Recovery" *Annals of Translational Medicine*, Vol. 11, No. 8, 30 April 2023 (2023-04-30), pages 1-15<br>page 1, “Methods”, “Results”, and “Conclusions” sections | 1-9 |
| X | GENG, Ruixuan et al. "α-Ionone Protects Against UVB-Induced Photoaging in Epidermal Keratinocytes" *Chinese Herbal Medicines*, 09 November 2022 (2022-11-09), pages 132-138<br>abstract, “Results” section | 1-9 |
| X | CN 1255367 A (SHISEIDO CO., LTD.) 07 June 2000 (2000-06-07)<br>description, page 1, paragraph 2 from the bottom, and page 2, paragraph 3 from the bottom, and tables 2 and 3 | 1-9 |
| A | Dabin Choi et al. "β-Ionone Attenuates Dexamethasone-Induced Suppression of Collagen and Hyaluronic Acid Synthesis in Human Dermal Fibroblasts" *Biomolecules*, 21 April 2021 (2021-04-21), pages 1-14<br>abstract | 1-9 |

☑ Further documents are listed in the continuation of Box C. ☑ See patent family annex.

* Special categories of cited documents:

- “A” document defining the general state of the art which is not considered to be of particular relevance
- “D” document cited by the applicant in the international application
- “E” earlier application or patent but published on or after the international filing date
- “L” document which may throw doubts on priority claim(s) or which is cited to establish the publication date of another citation or other special reason (as specified)
- “O” document referring to an oral disclosure, use, exhibition or other means
- “P” document published prior to the international filing date but later than the priority date claimed
- “T” later document published after the international filing date or priority date and not in conflict with the application but cited to understand the principle or theory underlying the invention
- “X” document of particular relevance; the claimed invention cannot be considered novel or cannot be considered to involve an inventive step when the document is taken alone
- “Y” document of particular relevance; the claimed invention cannot be considered to involve an inventive step when the document is combined with one or more other such documents, such combination being obvious to a person skilled in the art
- “&” document member of the same patent family

| Date of the actual completion of the international search | Date of mailing of the international search report |
|---|---|
| **01 March 2024** | **13 March 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

INTERNATIONAL SEARCH REPORT

International application No.
PCT/CN2023/137492

C. DOCUMENTS CONSIDERED TO BE RELEVANT

| Category* | Citation of document, with indication, where appropriate, of the relevant passages | Relevant to claim No. |
|---|---|---|
| A | KR 20160066186 A (AMOREPACIFIC CORP.) 10 June 2016 (2016-06-10)<br>claims 1-6 | 1-9 |
| A | US 2004131648 A1 (THE PROCTER & GAMBLE COMPANY) 08 July 2004 (2004-07-08)<br>description, paragraphs [0023]-[0026] | 1-9 |
| A | CN 109348703 A (INDUSTRY-ACADEMIC COOPERATION FOUNDATION, YONSEI UNIVERSITY) 15 February 2019 (2019-02-15)<br>claims 1-12 | 1-9 |

Form PCT/ISA/210 (second sheet) (July 2022)

**INTERNATIONAL SEARCH REPORT**
**Information on patent family members**

International application No.
**PCT/CN2023/137492**

| Patent document cited in search report | Publication date (day/month/year) | Patent family member(s) | Publication date (day/month/year) |
|---|---|---|---|
| CN 1255367 A | 07 June 2000 | DE 69930603 D1 | 18 May 2006 |
| | | DE 69930603 T2 | 12 October 2006 |
| | | EP 0988856 A2 | 29 March 2000 |
| | | EP 0988856 A3 | 14 November 2001 |
| | | EP 0988856 B1 | 29 March 2006 |
| | | HK 1029049 A1 | 23 March 2001 |
| | | KR 20000023421 A | 25 April 2000 |
| | | KR 100595737 B1 | 03 July 2006 |
| | | TW 550085 B | 01 September 2003 |
| | | US 6315980 B1 | 13 November 2001 |
| | | CN 1160087 C | 04 August 2004 |
| KR 20160066186 A | 10 June 2016 | KR 102298799 B1 | 07 September 2021 |
| US 2004131648 A1 | 08 July 2004 | JP 2006507287 A | 02 March 2006 |
| | | AU 2003285042 A8 | 13 May 2004 |
| | | CA 2500974 A1 | 06 May 2004 |
| | | WO 2004037213 A2 | 06 May 2004 |
| | | WO 2004037213 A3 | 29 July 2004 |
| | | EP 1553916 A2 | 20 July 2005 |
| | | CN 1705468 A | 07 December 2005 |
| CN 109348703 A | 15 February 2019 | KR 101702389 B1 | 03 February 2017 |
| | | WO 2017222317 A1 | 28 December 2017 |
| | | US 2017367954 A1 | 28 December 2017 |
| | | US 10137070 B2 | 27 November 2018 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- *Molecules*, 2019, vol. 24 (9), 1804
- Food Funct.. The Royal Society of Chemistry, 05 February 2019
- *Science and Technology of Food Industry*, 2022, vol. 43 (20), 481-488